(19) Europäisches Patentamt
European Patent Office
Office européen des brevets

(11) Publication number: **0 127 313**
**B1**

# (12) EUROPEAN PATENT SPECIFICATION

(45) Date of publication of patent specification: **19.07.89**

(51) Int. Cl.[4]: **A 01 N 43/40,** A 01 N 43/58, A 01 N 61/00

(21) Application number: **84302772.3**

(22) Date of filing: **25.04.84**

## (54) The production of haploid seed, of doubled haploids and of homozygous plant lines therefrom.

(30) Priority: **26.04.83 US 488888**
**28.02.84 US 584358**

(43) Date of publication of application:
**05.12.84 Bulletin 84/49**

(45) Publication of the grant of the patent:
**19.07.89 Bulletin 89/29**

(84) Designated Contracting States:
**AT BE CH DE FR GB IT LI LU NL SE**

(56) References cited:
**EP-A-0 037 133**
**EP-A-0 037 134**
**EP-A-0 040 082**
**US-A-4 115 101**
**US-A-4 345 934**

(73) Proprietor: **CENTRE NATIONAL DE LA RECHERCHE SCIENTIFIQUE (CNRS)**
**15, Quai Anatole France**
**F-75007 Paris (FR)**

(73) Proprietor: **INSTITUT NATIONAL DE LA RECHERCHE AGRONOMIQUE (INRA)**
**145, rue de l'Université**
**F-75341 Paris Cédéx 07 (FR)**

(73) Proprietor: **UNIVERSITE PARIS-SUD (PARIS XI)**
**15, rue Georges Clémenceau**
**F-91405 Orsay Cédex (FR)**

(72) Inventor: **Sherba, Samuel Eugene**
**41 Montclair Lane**
**Willingboro New Jersey 08046 (US)**
Inventor: **Warner, Harlow Lester**
**111 Quince Drive**
**Hatboro Pennsylvania 19040 (US)**

(74) Representative: **Martin, Jean-Jacques et al**
**Cabinet REGIMBEAU 26, Avenue Kléber**
**F-75116 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention is concerned with the production of haploid seed, of doubled haploids and homozygous plant lines therefrom.

Haploid and doubled haploid, including polyploids, angiosperms are known to occur spontaneously. Occurrences of haploid angiosperms have been recorded in at least seventy-one species, representative of thirty-nine genera in sixteen different families. Kimber et al., *Bot, Rev.,* 29:480—531 (1963). However, the spontaneous origin of haploids, whether it be by monoembryony or polyembryony of parthenogenic or androgenic origin, is unpredictable and occurs at a very low frequency. In an attempt to increase their frequency of occurrence, many techniques have been developed to induce haploidy in angiosperms. Examples of such techniques include emasculation and isolation, delayed pollination, pseudogamy by the use of abortive pollen or distant hybridization, semigamy, cytoplasmgenome interactions by chromosome elimination and alloplasmy, genetic selection techniques, culture methods, including anther culture, pollen culture and protoplast or tissue culture of haploids, and treatments by physical agents such as traumatic injuries, temperature shocks, x-rays, gamma-rays, radioisotopes and ultraviolet light radiations, and chemical agents. Examples of such chemicals include colchicine, maleic hydrazide, belbitan, parafluorophenylalanine, toluidene blue, nitrous oxide and chloramphenicol. A review of these techniques is given by Raghavan, Z., *Experimental Embryogenesis in Vascular Plants,* Chapter 15, Academic Press, 1976 and by Lacadena, J., "Spontaneous and Induced Parthenogensis and Androgensis" which is a chapter in Kasha, K.J. (editor), *Haploids in Higher Plants* : *Advances and Potential,* University of Guelph, Ontario, 1974.

However, all of these methods of inducement are very tedious, unpredictable and most result in extremely low frequency of occurrence of haploids from any given stock. Although the culture techniques may enable a greater frequency of occurrence, they are not suitable for a number of species and are, in any event, fairly tedious, require sophisticated series of cultures to develop an embryo and ultimately a plant and may require inducement for the doubling of the haploid. Few of these techniques, and none using a chemical agent, have resulted in sufficient or consistent occurrence of haploids or doubled haploids to enable them to be utilized readily in the development of plant breeding materials or breeding programs.

Additionally, abnormal wheat seed having haploidy properties has been observed after the treatment of wheat plants with a chemical hybridizing agent. Embryos isolated from these seeds were grown into plantlets on a culture utilized for the growth of haploid plants from androgenetic embryos.

We have now found that haploid angiosperms can be obtained by treating plants with an apomixic agent between the occurence of meiosis and anthesis, allowing the plants to develop seed, collecting the seed having characteristics of haploid seed prior to its abortion and maintaining the seed under conditions which preserve the viability of the embryo contained in the seed. A doubled haploid is produced from the haploid by supplying an environment which fosters and maintains the growth of the embryo and its development into a doubled haploid plant. The completion of the doubling process is grossly observed in those of the flowers which set seed. These seeds are genetically stable and each seed produced represents a single seed source of a homozygous line.

The present invention recognises that the timing and dosage of application of a chemical apomixic agent is important in obtaining haploids and doubled haploids, enabling their production in large numbers. The apomixic technique of the present invention is useful in plant breeding for obtaining genetically stable lines, thereby reducing or eliminating the need for inbreeding of plant lines. The obtaining of a homozygous plant can be accomplished within one generation Thus, it is a useful technique for the development of new varieties and germplasms. Additionally, due to the large numbers of doubled haploids which can be produced from the invention, it has utility in the development phase of breeding programs because it will allow a breeder to fix the genetic system of individual gametes in testable, reproducible form at any stage in a breeding program. Moreover, to the extent doubled haploid plants represent random gametes from their source material, the technique can be useful in inheritance studies because superior gametes will have the same opportunity as other gametes of being included in any sample of a gametic population obtained via haploids. Further the technique can be useful in the determination of phylogenetic relationships and genetic ratios.

For the purposes of this invention the following terms have the definitions given below.

"Haploid" is an organism that has a gametic chromosome complement (n). It includes monoploids (x), diploids (2x) and polyploids. The haploid may be of male and/or famale origin.

"Doubled haploid" is an organism whose gametic chromosome complement has been doubled (2n).

"Apomixis" is the reproduction of an angiosperm where the sexual organs or related structures take part, but where embryos are formed without the normal union of the male and female gamete to form a zygote. The specific origin of the apomixic haploids produced by this invention is not known and is not critical to the invention. For example, the apomixic haploids may originate from parthenogenesis, androgenesis, apogamety, semigamy or by chromosomal elimination after the fusion of male and female gametes.

The angiosperm plants treated to produce the haploids and doubled haploids may be either monocotyledons or dicotyledons which are either homozygous or heterozygous. The plants may be monoploid, diploid of polyploid. The invention is particularly applicable to commercially valuable crops

and seeds, especially cereal crops, such as wheat, barley, corn, rice, sorghum, millets, triticale, oats and rye and forage crops, such as rye grass, bent grass, fescue, timothy and orchard grass.

The apomixic agent is a chemical having the ability to cause apomixis in angiosperms. It may be one or more of the following: oxo and thio pyridazine such as those disclosed in European Patent Application 81304487, US—A 4345934 and Japanese Application 81/156321 and European Patent Application 83301057; 4-oxo-nicotinates (pyrid-4-ones) (and thio analogs) such as those disclosed in European Patent Application 81302079, in U.S. 4,115,101; U.S. 4,051,142; U.S. 3,503,986; U.S. 4,028,084; and U.S. 4,238,220; and pyrimidones such as those disclosed in U.S. 4,147,528, all of which are plant growth regulators. The oxo or thio pyridazines and the phenyl-substituted 4-oxo(thio)nicotinates are preferred. Chemical hybridizing agents are preferred apomixic agents, although such compounds are generally ineffective as hybridizing agents when applied at or after meiosis, the preferred time of application when used as apomixic agents.

The preferred phenyl-substituted-4-oxo(thio)nicotinates have the following structure:

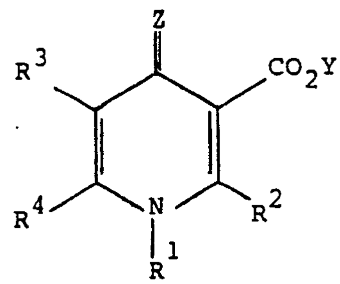

wherein

Z is an oxygen or sulfur atom;

$R^1$ is an optionally substituted ($C_1$—$C_6$)alkyl or ($C_2$—$C_6$)alkenyl; at least one of $R^2$, $R^3$ or $R^4$ is a

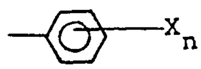

("the phenyl group") wherein

when $R^2$ is "the phenyl group", $R^3$ is a hydrogen atom, an alkyl group or a halogen atom and $R^4$ is a hydrogen atom, an alkyl group or independently "the phenyl group";

when $R^3$ is "the phenyl group", $R^2$ is an alkyl group or independently "the phenyl group", and $R^4$ is a hydrogen atom or an alkyl group;

when $R^4$ is "the phenyl group", $R^2$ is an optionally substituted ($C_2$—$C_6$)alkyl group, ($C_3$—$C_6$)alkenyl group or, independently, "the phenyl group" and $R^3$ is a hydrogen atom, a ($C_1$—$C_6$)alkyl group or a halogen atom; and

Y is a hydrogen atom, an agronomically acceptable salt thereof or an alkyl group;

X is a hydrogen atom, a halogen atom, a trihalomethyl group, a ($C_1$—$C_6$)alkyl group, a nitro group, a cyano group or a ($C_1$—$C_6$)alkoxy group; and

n is an integer of from 1 to 3.

Preferably Z is an oxygen atom; $R^1$ is a ($C_1$—$C_4$)alkyl group, more preferably a methyl, ethyl or n-propyl group; either $R^2$ or, more preferably, $R^4$ is "the phenyl group":

when $R^2$ is "the phenyl group", $R^3$ is a ($C_1$—$C_3$)alkyl, bromine or, more preferably, a hydrogen atom; $R^4$ is a hydrogen atom, a halogen atom or a ($C_1$—$C_4$)alkyl group, more preferably a ($C_1$—$C_3$)alkyl group;

when $R^4$ is "the phenyl group", $R^2$ is a hydrogen atom, a halogen atom or, more preferably, a ($C_1$—$C_4$)alkyl group; $R^3$ is a halogen atom or, more preferably, a hydrogen atom;

Y is a hydrogen atom, ($C_1$—$C_6$)alkyl preferably ($C_1$—$C_4$)alkyl, or an alkali metal or alkaline earth metal cation, more preferably a hydrogen atom, a methyl group, ethyl group, a sodium cation or a potassium cation;

X is a hydrogen atom, a halogen atom, a trifluoromethyl group or a ($C_1$—$C_4$)alkyl group, more preferably a hydrogen, chloro, bromo, fluoro or iodo atom, at the 3 and/or 4 position; and

n is 1 or 2.

The most preferred nicotinates are where Z is an oxygen atom; $R^1$ is a methyl or ethyl group; $R^3$ is a hydrogen atom; $R^2$ or, more preferably, $R^4$ is "the phenyl group"; Y is a sodium or potassium cation; X is hydrogen, chloro or fluoro atom at the 3 and/or 4 position.

Examples of such preferred 4-oxonicotinates include:

ethyl 1,2-dimethyl-6-(4-chlorophenyl)-4-oxonicotinate acid;

ethyl 1,2-dimethyl-6-phenyl-4-oxonicotinate acid, and the sodium and potassium salts of:

1-ethyl-2-(4-chlorophenyl)-6-methyl-4-oxonicotinic acid;

1-ethyl-2-(3,4-dichlorophenyl)-6-methyl-4-oxonicotinic acid;

1-ethyl-2-(3-chlorophenyl)-6-methyl-4-oxonicotinic acid;

1-methyl-2-(3-chlorophenyl)-6-methyl-4-oxonicotinic acid;

1-ethyl-2-(4-fluorophenyl)-6-methyl-4-oxonicotinic acid;
1-methyl-2-(4-chlorophenyl)-6-methyl-4-oxonicotinic acid;
1-ethyl-2-(3-fluorophenyl)-6-methyl-4-oxonicotinic acid;
1-methyl-2-(3-fluorophenyl)-6-methyl-4-oxonicotinic acid;
1,2-dimethyl-6-(4-chlorophenyl)-4-oxonicotinic acid;
1-methyl-2-*n*-propyl-6-(4-chlorophenyl)-4-oxonicotinic acid;
1,2-dimethyl-6-(3-chlorophenyl)-4-oxonicotinic acid;
1-methyl-2-*n*-propyl-6-phenyl-4-oxonicotinic acid;
1-ethyl-2-methyl-6-(4-chlorophenyl)-4-oxonicotinic acid;
1-*n*-propyl-2-methyl-6-phenyl-4-oxonicotinic acid;
1,2-dimethyl-6-(4-trifluoromethylphenyl)-4-oxonicotinic acid;
1,2-dimethyl-6-(4-fluorophenyl)-4-oxonicotinic acid;
1,2-dimethyl-2-methyl-6-phenyl-4-oxonicotinic acid; and

The preferred 4-oxo- or thio-pyridazines have the following structure:

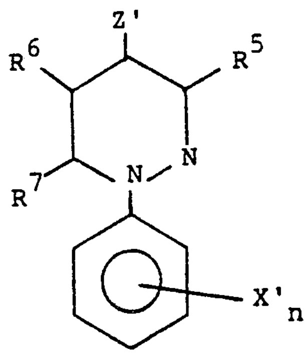

wherein

$Z^1$ is an oxygen or sulfur atom;

$R^5$ is a hydrogen atom, halogen atom,

$$-\overset{O}{\overset{\|}{C}}NRR$$

wherein R and R are each independently hydrogen, alkyl, alkyl substituted with a carbonyl or carboxy group,

$$-\overset{O}{\overset{\|}{C}}WR^8$$

wherein W is an oxygen or sulfur atom and $R^8$ is a hydrogen atom, alkyl group, aryloxy, alkoxyalkyl, alkylhalogen, alkylhydroxy, iminoalkyl (—N=alkyl), aryl, alkenyl, cyclo($C_3$—$C_6$)alkyl or cycloalkyl alkyl group;

$R^6$ is a hydrogen atom, alkyl,

$$-\overset{O}{\overset{\|}{C}}A$$

wherein A is hydrogen, alkyl, aralkyl, hydroxy, alkoxy, thio, alkylthio or $-NR^9R^{10}$ wherein $R^9$ and $R^{10}$ are each independently hydrogen, alkyl, alkyl substituted with a carbonyl or carboxy group, provided that $R^5$ and $R^6$ are not both hydrogen atoms; and when $R^5$ is a hydrogen or halogen atom, then $R^6$ is

$$-\overset{O}{\overset{\|}{C}}A'$$

wherein A' is an alkoxy, thioalkyl or $-NR^9R^{10}$ group; and

$R^7$ is alkyl, aryl or aryl substituted with up to three substituents selected from the group consisting of halogen, nitro, trihalomethyl, ($C_1$—$C_4$)alkoxyl ($C_1$—$C_4$)alkyl or cyano;

n is 0, 1, 2 or 3;

each X' is independently a hydrogen atom, halogen atom, trihalomethyl, alkyl, alkoxy, cyano or nitro group; and the agronomically acceptable salts thereof.

Preferably Z′ is an oxygen atom; $R^5$ is hydrogen, halogen or

$$-\overset{O}{\overset{\|}{C}}WR^8$$

wherein W is an oxygen atom and $R^8$ is a hydrogen atom, $(C_1—C_4)$alkyl, $(C_1—C_4)$alkylhalogen, $(C_1—C_4)$alkylhydroxy, $(C_1—C_4)$alkoxy$(C_1—C_4)$alkyl, $(C_1—C_2)$phenyl, $(C_1—C_4)$alkenyl or imino$(C_1—C_4)$alkyl group or W is sulfur and $R^5$ is a $(C_1—C_4)$alkyl group;

$R^6$ is hydrogen, $(C_1—C_4)$alkyl,

$$-\overset{O}{\overset{\|}{C}}A$$

wherein A is $(C_1—C_4)$alkyl, $(C_1—C_4)$alkoxy, $(C_1—C_4)$alkylthio or $—NR^9R^{10}$ and when $R^5$ is hydrogen or halogen, then $R^6$ is

$$-\overset{O}{\overset{\|}{C}}A$$

and A is $(C_1—C_4)$alkoxy or $(C_1—C_4)$alkylthio; $R^7$ is a $(C_1—C_4)$alkyl group; and X′ is a hydrogen, halogen or trifluoromethyl group at the 3 and/or 4 positions; n is 1 or 2; and the agronomically acceptable salts thereof.

More preferably Z′ is an oxygen atom; $R^5$ is

$$-\overset{O}{\overset{\|}{C}}WR^8,$$

wherein W is an oxygen atom and $R^8$ is a hydrogen atom (preferably a sodium, potassium or triethanolamine salt thereof), or a $(C_1—C_4)$alkyl (preferably a $(C_1—C_2)$alkyl) group; $R^6$ is hydrogen or

$$-\overset{O}{\overset{\|}{C}}A'$$

wherein A′ is hydroxy or $(C_1—C_4)$alkoxy (preferably a $(C_1—C_2)$alkoxy) group; $R^7$ is a methyl, ethyl or *n*-propyl group; and X′ is a chloro, bromo, fluoro or iodo atom, preferably chloro or bromo atom, at the 3 and/or 4 positions; n is 1 or 2; and the agronomically acceptable salts thereof.

Examples of preferred oxopyridazines include the following:

propen-1-yl 1-(4-chlorophenyl)-1,4-dihydro-4-oxo-6-methylpyridazine-3-carboxylate;
thiopropyl 1-(4-chlorophenyl)-1,4-dihydro-4-oxo-6-methylpyridazine-3-carboxylate;
triethanolamine 1-(4-chlorophenyl)-1,4-dihydro-4-oxo-6-methylpyridazine-3-carboxylate;
methyl 1-(4-chlorophenyl)-1,4-dihydro-4-oxo-6-ethylpyridazine-3-carboxylate;
methyl 1-(4-chlorophenyl)-1,4-dihydro-4-oxo-5-*n*-propyl-6-methylpyridazine-3-carboxylate;
methyl 1-(4-iodophenyl)-1,4-dihydro-4-oxo-6-methylpyridazine-3-carboxylate;
*n*-butyl 1-(4-bromophenyl)-1,4-dihydro-4-oxo-6-methylpyridazine-3-carboxylate;
methyl 1-(4-chlorophenyl)-1,4-dihydro-4-oxo-6-ethylpyridazine-5-carboxylate;
methyl 1-(4-chlorophenyl)-1,4-dihydro-3-bromo-4-oxo-6-ethylpyridazine-5-carboxylate;
methyl 1-(4-chlorophenyl)-1,4-dihydro-4-oxo-5-acetyl-6-ethylpyridazine-3-carboxylate;
sodium or potassium 1-(4-chlorophenyl)-1,4-dihydro-4-oxo-5-acetyl-6-ethylpyridazine-3-carboxylate;
sodium or potassium 1-(4-trifluoromethylphenyl)-1,4-dihydro-4-oxo-5-acetyl-6-ethylpyridazine-3-carboxylate;
sodium or potassium 1-(4-bromophenyl)-1,4-dihydro-4-oxo-5-acetyl-6-ethylpyridazine-3-carboxylate;
sodium or potassium 1-phenyl-1,4-dihydro-4-oxo-5-acetyl-6-ethylpyridazine-3-carboxylate;
sodium or potassium 1-(4-chlorophenyl)-1,4-dihydro-4-oxo-6-*n*-propylpyridazine-5-carboxylate;
1-(4-chlorophenyl)-1,4-dihydro-4-oxo-6-methylpyridazine-3-carboxylate acid;
sodium or potassium 1-(4-chlorophenyl)-1,4-dihydro-4-oxo-5-carboxymethyl-6-ethylpyridazine-3-carboxylate;
sodium or potassium 1-(3,4-dichlorophenyl)-1,4-dihydro-4-oxo-5-carboxymethyl-6-ethylpyridazine-3-carboxylate;
1-(3,4-dichlorophenyl)-1,4-dihydro-4-oxo-5-carboxymethyl-6-ethylpyridazine-3-carboxylic acid;
1-(4-chlorophenyl)-1,4-dihydro-3,5-dicarboxymethyl-4-oxo-6-ethylpyridazine;

sodium or potassium 1-(4-iodophenyl)-1,4-dihydro-4-oxo-5-carboxymethyl-6-ethylpyridazine-3-carboxylate;

1-(4-iodophenyl)-1,4-dihydro-3,5-dicarboxymethyl-4-oxo-6-ethylpyridazine;

sodium or potassium 1-(2-chlorophenyl)-1,4-dihydro-4-oxo-5-carboxymethyl-6-ethylpyridazine-3-carboxylate;

sodium or potassium 1-(4-chlorophenyl)-1,4-dihydro-4-oxo-5-carboxymethyl 6-*n*-propylpyridazine-3-carboxylate;

1-(3,4-dichlorophenyl)-1,4-dihydro-3,5-dicarboxymethyl-4-oxo-6-ethylpyridazine;

sodium or potassium 1-(4-bromophenyl)-1,4-dihydro-4-oxo-5-carboxymethyl-6-ethylpyridazine;

1-(4-bromophenyl)-1,4-dihydro-3,5-dicarboxymethyl-4-oxo-6-ethylpyridazine;

1-(4-chlorophenyl)-1,4-dihydro-3,5-dicarboxymethyl-4-oxo-6-ethylpyridazine;

Generally, the 4-pyridazinones are more preferred as apomixic agents than the phenyl-substituted-4-oxo-nicotinates.

As utilized in the present invention, the term "alkyl" is meant to include straight or branched chain alkyl groups, preferably of up to 4 carbon atoms. The term "aryl" is meant to include naphthyl, substituted naphthyl groups or, preferably, phenyl or phenyl substituted groups. When the aryl is substituted, it has up to three substituents, preferably up to two substituents, selected from the group consisting of halogen, nitro, trihalomethyl, $(C_1—C_4)$alkoxy, $(C_1—C_4)$alkyl and cyano.

Since the compounds utilized in the present invention possess both acidic and basic functional groups, the term "agronomically acceptable salts" is meant to include salts of the carboxyl group such as alkali metals, alkaline earth metals or transitional metals, ammonium or substituted ammonium groups as well as acid addition salts such as hydrochloride, hydrobromide, sulfate, nitrate, perchlorate, acetate, oxalate and the like. Representative metal salt cations include alkali metal cations which are preferred, such as sodium, potassium, lithium, or the like, alkaline earth metal cations, such as calcium, magnesium, barium, strontium or the like, or heavy metal cations, such as zinc, manganese, cupric, cuprous, ferric, ferrous, titanium, aluminum, or the like.

Among the ammonium salts are those of the formula

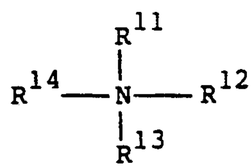

in which $R^{11}$, $R^{12}$, $R^{13}$ and $R^{14}$ are individually a hydrogen atom, a hydroxy group, a $(C_1—C_4)$alkoxy group, a $(C_1—C_{20})$alkyl group, a $(C_3—C_8)$alkenyl group, a $(C_3—C_8)$alkynyl group, a $(C_2—C_8)$hydroxy alkyl group, a $(C_2—C_8)$alkoxyalkyl group, a $(C_2—C_6)$aminoalkyl group, a $(C_2—C_6)$haloalkyl group, a substituted or unsubstituted phenyl group, a substituted or unsubstituted phenylalkyl group, having up to 4 carbon atoms in the alkyl moiety, an amino or alkyl-substituted amino group, or any two of $R^{11}$, $R^{12}$, $R^{13}$ or $R^{14}$ can be taken together to form with the nitrogen atom a 5- or 6-member heterocyclic ring, optionally having up to one additional hetero oxygen, nitrogen, or sulfur atom in the ring, and preferably saturated, such as piperidine, morpholine, pyrrolidine or piperazine ring, or the like, or any three of $R^{11}$, $R^{12}$, $R^{13}$ or $R^{14}$ can be taken together to form with the nitrogen atom a 5- or 6-member optionally substituted aromatic heterocyclic ring, such as pyrrole, pyridine, nicotine or histamine. When the ammonium group contains a substituted alkyl, substituted phenyl, or substituted phenylalkyl group, the substituents will generally be selected from halogen atoms, $(C_1—C_8)$alkyl groups, $(C_1—C_4)$-alkoxy groups, hydroxy groups, nitro groups, trifluoromethyl groups, cyano groups, amino groups, $(C_1—C_4)$alkylthio groups, and the like. Such substituted phenyl groups preferably have up to two such substituents. Representative ammonium cations include ammonium, dimethylammonium 2-ethylhexyl- ammonium, bis(2-hydroxyethyl)ammonium, *t*-octylammonium; 2-hydroxyethylammonium, morpholinium, piperidinium, 2-phenethylammonium, 2-methylbenzyl-ammonium, *n*-hexylammonium, triethylammonium, trimethylammonium, tri(n-butyl)ammonium, methoxy-ethylammonium, diisopropylammonium, pyridinium, diallylammonium, pyrazolium, propargylammonium, dimethyl-hydrazinium, hydroxyammonium, methoxyammonium, dodecylammonium, octadecylammonium, 4-dichlorophenylammonium, 4-nitrobenzylammonium, benzyltrimethylammonium, 2-hydroxyethyldimethyloctadecylammonium, 2-hydroxyethyldiethyloctylammonium, decyltrimethylammonium, hexyltriethylammonium, 4-methylbenzyltrimethylammonium, and the like.

The species of plant being treated will determine the particular apomixic agent(s) which may be utilized. For example, an apomixic agent which can cause apomixis to occur in common wheat may not have an apomixic effect on corn or soybeans. Preferred apomixic agents for producing haploids of *Graminaeae* plants, particularly small grains such as wheat, barley, oats, rye, etc. include the following and, preferably their water soluble salts: 1-(4-chlorophenyl)-1,4-dihydro-4-oxo-6-methylpyridazine-3-carboxylic acid, 1-(4-chlorophenyl)-1,4-dihydro-4-oxo-5-carboxymethyl-6-ethylpyridazine-3-carboxylic acid, 1-(4-chlorophenyl)-1,4-dihydro-4-oxo-5-propionyl-6-methylpyridazine-3-carboxylic acid, 1-ethyl-6-methyl-2-(4-chlorophenyl)-4-oxonicotinic acid, 1-ethyl-6-methyl-2-(3,4-dichlorophenyl)-4-oxonicotinic acid, 1-(3,4-

dichlorophenyl)-1,4-dihydro-4-oxo-5-carboxymethyl-6-methylpyridazine and 1-phenyl-1,4-dihydro-4-oxo-5-carboxymethyl-6-ethylpyridazine as well as methyl or ethyl 1-(4-chlorophenyl)-1,4-dihydro-4-oxo-6-ethylpyridazine-5-carboxylate and methyl or ethyl 1-(4-bromophenyl)-1,4-dihydro-4-oxo-6-ethylpyridazine-5-carboxylate. These compounds are particularly useful for inducing the occurrence of haploids and their subsequent doubled haploids in wheat.

In the application of the apomixic agent, the haploid seed is generally produced by a process which involves charging to a container or mechanical dissemination device, a composition comprising the apomixic agent and, usually, an agronomically acceptable carrier therefor. The thus charged container or mechanical dissemination device is then used to apply the apomixic agent to the plant, for example by the application of a dust or liquid spray to the plant foliage.

The apomixic agent is applied to a plant prior to the occurrence of fertilization. Generally, it is applied between the occurrence of meiosis and antheis. If the apomixic agent is applied prior to flower initiation, the agent is relatively less active and may cause plant injury. If the agent is applied after anthesis, haploids are not induced.

The apomixic agent is applied in an amount sufficient to cause apomixis and the induction of haploids. Frequently appropriate application rates are about 0.01 to about 22 kilograms per hectare, preferably from about 0.03 to about 11 kilograms and more preferably from about 0.1 to about 6 kilograms per hectare. The rate of application will vary depending upon the particular apomixic agent used, the particular plant species being treated and the stage of growth at treatment. Generally, the later the stage of growth, up to anthesis, the less apomixic agent required.

In order to apply apomixic agents to the plant, they may be applied foliarly, to the soil or, in rice crops, to the water surface. Foliar application is preferred.

Generally, some sort of stimulus, e.g., pollen, is beneficial in causing the plant to produce seed after treatment with the apomixic agent. If the apomixic agent is applied prior to the occurrence of meiosis, then pollen from another plant is useful in stimulating the plant to continue its reproductive cycle and produce seed. If the apomixic agent is applied after meiosis and the plant is a self-pollinator, then generally its own pollen is sufficient to stimulate seed production.

The apomixic seed is identified by characteristics which are the same as those for the specific species of haploid seed occurring naturally or induced by other means. Such characteristics include, for example, morphological ones and genetic markings. For example, the apomixic seed of common wheat is flatter and smaller, containing less or no endosperm, than normal seed produced by sexual reproduction. In those instances where the haploid seed is difficult to identify by morphological characteristics, separation may be effected by the use of genetic markers, e.g., of the seed or seedling stage or by flotation techniques, etc. as described in Lacadena, J., "Spontaneous and Induced Parthenogenesis and Androgenesis" occurring in Kasha, K. J. (editor), *Haploids in Higher Plants: Advances and Potential,* University of Guelph, Ontario, 1974 (see also the references cited therein). A haploid seed may also be identified by determining its chromosome count from a root tip taken from the embryo or plant developing from the seed.

The apomixic seed is collected prior to its abortion, while the seed contains a viable embryo. It is, therefore, usually collected within about four weeks after anthesis occurs, preferably between two and three weeks after anthesis.

After collection, the apomixic seed is treated in a manner to maintain the viability of the seed's embryo. Particularly in those instances where the haploid seed contains less endosperm than a normal seed of the same species, it is desirable to supply nutrients and other conditions which will foster and maintain the growth of the embryo and its subsequent development into a doubled haploid organism. This allows for a higher percentage of the haploid seeds to develop into doubled haploid plants. The use of particular nutrients and growth techniques can be readily determined by those in the art and include, for example, known nutrients such as a carbon source, minerals, amino acids, growth regulators, etc. and culture mediums such as wetted filter paper, agar, soil, etc. Such techniques are described, for example, in Raghavan, V., *Experimental Embryogenesis in Vascular Plants,* Academic Press, New York (1976). The use of aseptic conditions, the dissection of the embryo from the seed, temperature control, regulation of day length and vernalization or other physical conditions may also be utilized to enhance the growth and doubling of the haploid. If an embryo produced by the apomixic technique of this invention fails to spontaneously double its gametic chromosome complement or if it is desired to cause the doubling more readily or more uniformly, then a doubling agent, e.g., colchicine, may be used in accordance with techniques known to those in the art.

In one aspect, the present invention provides a means for obtaining homozygous lines within one generation from either a homozygous or heterozygous plant, thereby reducing the amount of time necessary for the development of germ plasms, new varieties and breeding material. For example, new homozygous lines may be obtained by subjecting a hybrid to the epomixic technique of the present invention, selecting and collecting the haploid seed, growing the embryo of the haploid seed under conditions favoring the develoment of a doubled haploid plant and collecting the seed produced from that plant. Even though the seed produced from the same and different plant will vary genetically, each seed will be genetically stable and represent a single seed source of homozygous line. Varieties, germ plasms and breeding materials are then obtained by selecting those plants which have the characteristics desired.

An apomixic agent of the invention can be applied to the growth medium or to plants to be treated

either by itself or, as is generally done, as a component in a composition or formulation which also comprises an agronomically acceptable carrier. By "agronomically acceptable carrier" is meant any substance which can be used to dissolve, disperse, or diffuse a compound in the composition without impairing the effectiveness of the compound and which by itself has no significant detrimental effect on the soil, equipment, crops or agronomic environment. Mixtures of the apomixic agents may also be used in any of these formulations. The compositions of apomixic agents can be either solid or liquid formulations or solutions. For example, the apomixic agents can be formulated as wettable powders, emulsifiable concentrates, dusts, granular formulations, aerosols, or flowable emulsion concentrates. In such formulations, the apomixic agents are extended with a liquid or solid carrier and, when desired, suitable surfactants are incorporated.

It is usually desirable, particularly in foliar applications, to include adjuvants, such as wetting agents, spreading agents, dispersing agents, stickers, adhesives, and the like, in accordance with agricultural practices. Examples of adjuvants which are commonly used in the art can be found in the John W. McCutcheon, Inc. publication "Detergents and Emulsifiers Annual".

The apomixic agents can be dissolved in any appropriate solvent. Examples of solvents which are useful in the practice of this invention include water, alcohols, ketones, aromatic hydrocarbons, halogenated hydrocarbons, dimethylformamide, dioxane, dimethyl sulfoxide, and the like. Mixtures of these solvents can vary from about 2% to about 98% by weight with a preferred range being from about 20% to about 75%.

For the preparation of emulsifiable concentrates, the apomixic agents can be dissolved in organic solvents, such as benzene, toluene, xylene, methylated naphthalene, corn oil, pine oil, o-dichlorobenzene, isophorone, cyclohexanone, methyl oleate, and the like, or in mixtures of these solvents, together with an emulsifying agent or surfactant which permits dispersion in water. Suitable emulsifiers include, for example, the ethylene oxide derivatives of alkylphenols or long-chain alcohols, mercaptans, carboxylic acids, and reactive amines and partially esterified polyhydric alcohols. Solvent-soluble sulfates or sulfonates, such as the alkaline earth salts or amine salts of alkyl-benzenesulfonates and the fatty alcohol sodium sulfates, having surface-active properties can be used as emulsifiers either alone or in conjunction with an ethylene oxide reaction product. Flowable emulsion concentrates are formulated similarly to the emulsifiable concentrates and include, in addition to the above components, water and a stabilizing agent such as a water-soluble cellulose derivative or a water-soluble salt of a polyacrylic acid. The concentration of the active ingredient in emulsifiable concentrates of usually about 10% to 60% by weight and in flowable emulsion concentrates, this can be as high as about 75%.

Wettable powders suitable for spraying, can be prepared by admixing the compound with a finely divided solid, such as clays, inorganic silicates and carbonates, and silicas and incorporating wetting agents, sticking agents, and/or dispersing agents in such mixtures. The concentration of active ingredients in such formulations is usually in the range of about 20% to 98% by weight, preferably about 40% to 75%. A dispersing agent may generally constitute about 0.5% to about 3% by weight of the composition, and a wetting agent may generally constitute from about 0.1% to about 5% by weight of the composition.

Dusts can be prepared by mixing the apomixic agents of the invention with finely divided inert solids which may be organic or inorganic in nature. Materials useful for this purpose include, for example, botanical flours, silicas, silicates, carbonates and clays. One convenient method of preparing a dust is to dilute a wettable powder with a finely divided carrier. Dust concentrates containing about 20% to 80% of the active ingredient are commonly made and are subsequently diluted to about 1% to 10% by weight use concentration.

Granular formulations can be prepared by impregnating a solid such as granular Fuller's earth, vermiculite, ground corn cobs, seed hulls, including bran or other grain hulls, or similar material. A solution of one or more of the apomixic agents in a volatile organic solvent can be sprayed or mixed with the granular solid and the solvent then removed by evaporation. The granular material can have any suitable size, with a preferable size range of 16 to 60 mesh. The active compound will usually comprise about 2 to 15% by weight of the granular formulations.

Salts of the apomixic agents can be formulated as aqueous formulations. The salt will typically comprise about 0.05 to about 75% by weight preferably about 10% to about 50%, of the aqueous formulation.

These compositions can also be further diluted with water if desired prior to actual application. In some applications, the activity of these compositions can be enhanced by incorporating into the composition an adjuvant such as glycerin, methylethyl-cellulose, hydroxyethyl-cellulose, polyoxyethylenesorbitan monooleate, polypropylene glycol, polyacrylic acid, polyethylene sodium malate, polyethylene oxide, or the like. The adjuvant will generally comprise about 0.1 to about 5% by weight, preferably about 0.5 to about 2% of the composition. Such compositions can also optionally include an agronomically-acceptable surfactant.

The apomixic agents can be applied as sprays by methods commonly employed, such as conventional hydraulic sprays, aerial sprays, and dusts. For low-volume applications, a solution of the compound is usually used. The dilution and volume of application will usually depend upon such factors as the type of equipment employed, the method of application, the area to be treated and the type and stage of development of the crop being treated.

The following Examples of some preferred embodiments of the invention are given for the purposes of illustration only.

## EXAMPLES

### Example 1

A spring wheat variety, *Fielder*, was grown under greenhouse conditions. A portion of the plants were treated with potassium 1-(4-chlorophenyl)-1,4-dihydro-4-oxo-6-methylpyridazine-3-carboxylate (apomixic agent) at dosage rates of about 0.28, 1.1, 2.2, and 4.4 kilograms (kg) of the apomixic agent per hectare (ha). (The apomixic agent was formulated with 0.25 volume/volume percent Triton® AG—98 low foam spray adjuvant which is a nonionic surfactant solution of octylphenoxypolyethoxy-ethanol and isopropanol). The apomixic agent was applied to the wheat at different growth stages, i.e., the initiation of jointing (Stage 6), second node detectable (Stage 7), flag leaf just visible (Stage 8), flag leaf ligule just visible (Stage 9), flag leaf half extended (Stage 9.5), flag leaf sheath fully extended (Stage 10), head or spike fully emergenced (Stage 10.5) and anthesis. At anthesis, selected spikes of these treated plants were cross pollinated with pollen from untreated wheat of a different line.

Two controls were utilized. One was *Fielder* plants which were not treated with the apomixic agent. The other control was similarly untreated and its flowers hand emasculated and cross pollinated with pollen from untreated wheat of a different line.

All the other flowers from both treated and untreated plants were allowed to self pollinate, if viable pollen were present.

Twenty days after pollination (anthesis), phototoxicity to the wheat spikes was determined and subjective observations were made on the ovary, embryo, and seed appearances. All of the ovaries were normal in appearance. The remainder of the results are given in Table I.

The seeds containing haploid embryos were shriveled and flat having a dull blue-green color compared to the control seeds which were plump having a shiny light green appearance. The seeds containing haploid embryos also contained watery endosperm which was lacking in starch and their embryos were generally larger and more developed than those of the control seeds.

Embryos from the haploid seeds were dissected from the seeds and placed on moist filter paper saturated with a sucrose solution where they germinated.

The morphological characteristics of the haploid seed, its embryo and plant which developed therefrom were typical of the characteristics which have been observed for naturally occurring wheat haploids.

TABLE 1

| Time of Treatment | Apomixic Agent kg/ha | Injury Rank[1] | SELF POLLINATED SPIKES Seed Appearance | SELF POLLINATED SPIKES Embryo Appearance | CROSS POLLINATED SPIKES Seed Appearance | CROSS POLLINATED SPIKES Embryo Appearance |
|---|---|---|---|---|---|---|
| Stage 6 | 0.28 | 0 | Normal | Normal | None | None |
| | 1.1 | 0 | Normal | Normal | None | None |
| | 2.2 | 3 | None | None | Blue-green, watery endosperm, slightly shriveled | Normal |
| | 4.4 | 5 | None | None | Blue-green, watery endosperm, slightly shriveled | Large |
| Stage 7 | 0.28 | 0 | Normal | Normal | Normal | Normal |
| | 1.1 | 1 | None | None | Blue green, watery endosperm, some starch | Large |
| | 2.2 | 5 | None | None | Blue green, watery endosperm, shriveled | Large |
| | 4.4 | 7 | None | None | None | None |
| Stage 8 | 0.28 | Trace | Blue-green Shriveled | Normal | Watery with ~50% starch | Normal |
| | 1.1 | 3 | None | None | Blue-green, shriveled | Large |
| | 2.2 | 5 | None | None | Blue-green, water shriveled | Large |
| | 4.4 | 7 | None | None | None | None |

TABLE 1 - (CONTINUED)

| Time of Treatment | Apomixic Agent kg/ha | Injury Rank[1] | SELF POLLINATED SPIKES Seed Appearance | SELF POLLINATED SPIKES Embryo Appearance | CROSS POLLINATED SPIKES Seed Appearance | CROSS POLLINATED SPIKES Embryo Appearance |
|---|---|---|---|---|---|---|
| Stage 9 | 2.2 | Trace | Green-tan, small shriveled | Normal | Green, slightly shriveled, water & starch large | Large |
| | 4.4 | 1 | Green, very small, Small shriveled | Small | Green-tan, shriveled, large | Large |
| Stage 9.5 | 1.1 | 0 | Green, watery, plump | Large | None | None |
| | 2.2 | 0 | Green-tan, watery, shriveled | Large | None | None |
| | 4.4 | 0 | Green-tan, watery shriveled | Large | None | None |
| Stage 10.0 | 1.1 | 0 | Green, slightly shriveled | Large | None | None |
| | 2.2 | 0 | Green-tan, watery, | Large | None | None |
| | 4.4 | 0 | Tan; shriveled, no starch | Large | None | None |
| Stage 10.5 | 4.4 | 0 | Tan, shriveled, no starch | Large | None | None |
| Anthesis | 4.4 | 0 | Brown, shriveled, no starch | Large | None | None |

TABLE 1 - (CONTINUED)

| Time of Treatment | Apomixic Agent kg/ha | Injury Rank[1] | SELF POLLINATED SPIKES Seed Appearance | SELF POLLINATED SPIKES Embryo Appearance | CROSS POLLINATED SPIKES Seed Appearance | CROSS POLLINATED SPIKES Embryo Appearance |
|---|---|---|---|---|---|---|
| Emasculated | 0.0 | 0 | None | None | Light green, shiney, plump, starchy | Normal |
| Control | 0 | 0 | Light green, shiney, plump, starchy | Normal | None | None |

1) Injury scale ranges from 0 to 9, wherein 0 indicates no injury and 9 indicates death.

Example 2

Spring wheat variety *Fielder* was grown under greenhouse conditions. A portion of the plants were treated with potassium 1-(4-chlorophenyl)-1,4-dihydro-4-oxo-6-methylpyridazine-3-carboxylate (apomixic agent) at about 0.6, 1.1, 2.2, and 4.4 kilograms of apomixic agent per hectare. (The apomixic agent was formulated with 0.4 volume/volume percent Triton® AG—98 low foam adjuvant). The apomixic agent was applied to the plants at times which averaged from three days prior to anthesis to four days after anthesis.

Three controls were utilized. One was *Fielder* plants which were not treated with any spray. The second control was sprayed with only 0.4 volume/volume percent Triton® AG—98 at three days before and after anthesis. The third set of control plants had its flowers hand emasculated and then cross pollinated with *Fielder* which was treated with 4.4 kilograms per hectare of the apomixic agent three days prior to anthesis.

Fourteen days after anthesis, seeds were collected from the florets of the spikes of the wheat plants and the average weight of the seeds was determined. The results are reported in Table 2.

Although some of the results are given as being after anthesis, some of these florets from which seed was obtained actually underwent anthesis after treatment with the apomixic agent. This was due to the fact that anthesis on the same spike does not occur in every floret on the same day.

TABLE 2

Average Seed Weight

| | Dosage | |
|---|---|---|
| Timing of Treatment | Apomixic Agent 0.6 kg/ha | 4.4 kg/ha |
| 3 days before anthesis | 11.3 mg/seed | 2.5 mg/seed |
| 2 days before anthesis | 28.7 | 2.5 |
| 1 day before anthesis | 28.3 | 3.9 |
| Anthesis | 21.7 | 4.4 |
| 1 day after anthesis | 35.1 | 2.6 |
| 2 days after anthesis | 36.1 | 25.3 |
| 3 days after anthesis | 42.7 | 38.7 |
| 4 days after anthesis | | 47.0 |

*Control Seeds*

No treatment: 45.1 mg/seed

Treated with only Triton AG—98 low foam adjuvant: 43.4 mg/seed

Hand emasculated plants crossed with pollen from plants treated with the apomixic agent 3 days before anthesis: 44.1 mg/seed

The lower weight is indicative of haploid seed.

Example 3

Different hard red winter wheats (third generation from their original crosses) were vernalized at 2°C for fifty days and then planted in six inch pots and grown in a greenhouse until the plants varied in growth from stage 9.0 to late boot (about stage 10). (Stages in this example refer to the commonly used and known Feekes scale, Feekes, W., "De tarwe en haar milieu" *Versl. techn. Tarwe Comm. 12:*523—888 (1941).) At this time, the plants were sprayed with potassium 1-(4-chlorophenyl)-1,4-dihydro-4-oxo-6-methylpyridazine-3-carboxylate (apomixic agent) at dosage rates of about 1.1, 2.2 and 3.3 kilograms (kg) of the apomixic agent per hectare (ha). Each tiller of each plant was tagged and identified as to its growth stage at the time of treatment. About two weeks after the treatment, anthesis occurred with seed set occurring in some of the heads.

About twenty-one days after anthesis, immature seeds having the characteristics of apomixic seed, e.g., dimpled seeds having irregular scutella and endosperms which were clear and liquidy, were removed from the heads, the embryos removed from these seeds and placed on Miller's soybean callus medium, a nutrient sterile agar containing indole acetic acid and salts and allowed to grow under room conditions.

The embryos which grew and developed roots and shoots were transplanted to test tubes containing fresh Miller's medium. The tubes containing the embryos were placed in an environmental chamber at about 70°F (21°C) and exposed to fourteen hours of daylight and ten hours of darkness to allow for their further development. After the plantlets developed good roots and about a three inch shoot, they were placed in a vernalizing chamber at 2°C for fifty days. Upon removal from the vernalizing chamber, the plantlets were potted in soil and grown under greenhouse conditions for about five days. At this time, root tip samples were taken from each plant to obtain chromosome counts.

The soil was brushed and rinsed (with water) from the root tip samples. The root tips from each plant were placed in a vial about one-third full of a saturated aqueous solution of 1-bromonaphthalene. The roots were left in the bromonaphthalene solution for about 3 to 4 hours. The roots were then transferred to another set of vials containing approximately 1 milliliter of 3 parts absolute ethyl alcohol and 1 part glacial acetic acid and left there for a little more than an hour. They were then transferred to another set of vials containing 5N hydrochloric acid for about 5½ to about 7 minutes. After which the roots were removed and washed in distilled water and placed in another set of vials containing a small amount of feulgen stain. (The feulgen stain was prepared by pouring 100 milliliters of boiling water over 0.5 grams of basic fuchsin in a flask and shaking for a few minutes until dissolved. The solution was then cooled to about 50°C and filtered into a brown stock bottle and 15 milliliters of 1N hydrochloric acid was added. The solution was cooled further to about 28°C and about 1.5 grams of potassium metabisulfate was added and was dissolved with shaking. After refrigerating overnight, about 1 to 2 tablespoons of activated decolorizing charcoal were added. The solution was shaken well and filtered into another brown stock bottle.) The vials were corked and stored in a refrigerator until the chromosome counts were taken.

Each of the root tips was removed from the feulgen-containing vials, placed on a glass slide and approximately half of the stained areas were cut off. A drop of acetocarmine stain was placed on the remaining sample and a cover slip was placed over each sample. The roots were squashed by placing gentle pressure on the cover slip. The slides were then viewed under a microscope and the chromosomes of single cells were counted.

In addition to doing a chromosome count on the above lines of seeds which were treated with the apomixic agent, chromosome counts were also taken on untreated lines. These seeds were placed in petri dishes on a piece of moist blotter paper with the germ side up. The seeds were allowed to germinate at room temperature for about 48 hours until the primary root was about ½ inch long. Samples of the primary roots of each plant were taken and chromosomal counts conducted as above described. The results of the chromosome counts are given below in Table 3. (No results were obtained on untreated seed line 5 because no cells were observed having countable chromosomes.)

Since the normal chromosome count of a wheat cell of the hard red winter wheat lines tested is 42, the obtainment of chromosome counts of 21 are very indicative of haploid wheat plants and those counts between 21 and 42 are very indicative that the chromosomes are undergoing endoreduplication, which upon completion, for example, at a later stage of the plant's development, results in doubled haploid plants.

TABLE 3

| Seed Line | Apomixic Agent (lb./acre) | Treatment Stage | Chromosome Count per cell(s) per plant |
|---|---|---|---|
| 1 | 0 | -- | 42, 42, 42, 42 |
| 1 | 2 | Early Boot | 28, 35, 28 |
| 1 | 1 | Early Boot | 21, 25, 21, 28 |
| 2 | 0 | -- | 42, 42 |
| 2 | 2 | Early Boot | 21, 28, 21, 42, 42, 28 |
| 2 | 2 | Early Boot | 21, 21, 28 |
| 2 | 2 | Early Boot | 21, 21 |
| 2 | 2 | Early Boot | 21, 21, 21, 28 |
| 4 | 0 | -- | 42, 42, 42 |
| 4 | 2 | Late Boot | {21, 42, 42, 37, 28, 28 |
| | | | {28, 21, 25, 20, 21 |
| 4 | 2 | Late Boot | 30, 28, 28 |
| 4 | 2 | Late Boot | 42, 35 |
| 4 | 2 | Late Boot | 28, 42 |
| 4 | 2 | Late Boot | 28, 28 |
| 4 | 3 | 9.0 | 28, 28, 21, 21, 21, 21 |
| 4 | 3 | 9.2 | 21, 28, 28, 21, 28 |
| 4 | 3 | 9.2 | 35, 21, 21 |
| 4 | 3 | Mid Boot | 42, 42 |
| 5 | 2 | 9.4 | 21, 28 |
| 5 | 2 | 9.4 | 21, 21, 40 |
| 5 | 2 | 9.4 | 21 |
| 5 | 2 | 9.5 | 42, 28 |
| 6 | 0 | -- | 42 |
| 6 | 2 | 9.0 | 28, 21, 21 |
| 6 | 2 | 9.6 | 21, 20, 21, 21, 21 |
| 6 | 2 | 9.6 | 42, 21, 28 |
| 6 | 2 | 9.6 | 21 |
| 6 | 2 | 9.6 | 21 |
| 6 | 2 | 9.6 | 21 |
| 6 | 2 | 9.6 | 21 |
| 6 | 2 | 9.6 | 21 |
| 6 | 2 | 9.6 | 35, 27, 24 |
| 9 | 0 | -- | 42, 42 |
| 9 | 3 | 9.2 | 28, 28 |
| 9 | 3 | 9.2 | 21, 21, 21, 28 |
| 9 | 3 | 9.2 | 28, 21, 21 |
| 10 | 0 | -- | 42, 42 |
| 10 | 2 | 9.2 | 21, 28 |

Apomixic agents useful in the present invention can be prepared by various synthetic routes found in the art. For example, the phenyl-substituted-4-oxo(thio)nicotinates can be prepared as follows when Z, $R^1$, Y, X and n are as defined above, and:

(1) when $R^2$ is the phenyl group, $R^3$ is a hydrogen atom, alkyl group, a halogen atom or the phenyl group, $R^4$ is hydrogen or an alkyl group, the compounds can be prepared by the reaction of a suitably substituted 4-hydroxy-2-pyrone of the formula:

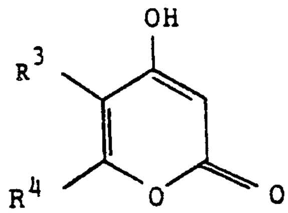

(III)

wherein $R^4$ is as defined above with a benzoylhalide of the formula:

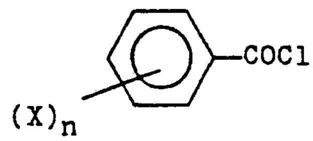

(IV)

wherein X is as defined above in the presence of an acid scavenger such as pyridine, triethylamine and the like, at temperatures from about 0 to about 10°C to form a 4-benzoyloxy-2-pyrone of the formula:

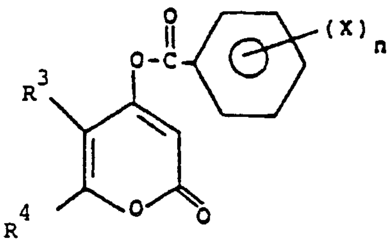

(V)

wherein $R^4$ and X and n are as defined above.

This reaction is discussed in E. Marcus, J. F. Stephen, J. K. Chan, *Journal of Heterocyclic Chemistry,* p. 13, 1966. This benzoate can undergo a Fries-type rearrangement with anhydrous aluminum chloride at elevated temperatures to give the product of the formula:

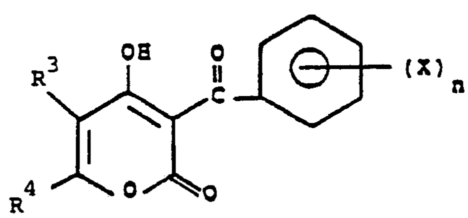

(VI)

as discussed in the E. Marcus, et al. reference, *ibid.* The benzoylpyrone of the formula (VI) above can then be reacted with a suitable alcohol (ROH) (wherein R is an alkyl) in the presence of a similarly substituted trialkylorthoformate $(RO)_3CH$ utilizing an acid catalyst selected from the group consisting of sulfuric, hydrochloric, trifluoroacetic, acetic, hydrobromic, and the like, at temperatures from about 0 to about 200°C to form the 3-carboxy-4-pyrone of the formula:

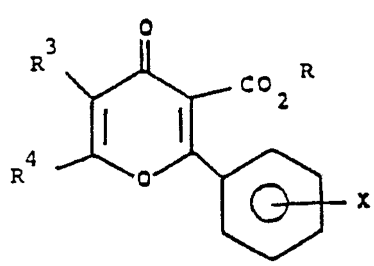

(VII)

The 3-carboxy-4-pyrone esters of formula (VII) can be reacted with any suitably substituted amine of the formula:

$$R^1—NH_2 \quad (VIII)$$

to yield a 1-alkyl-2-aryl-4-oxonicotinate ester of formula (IX).

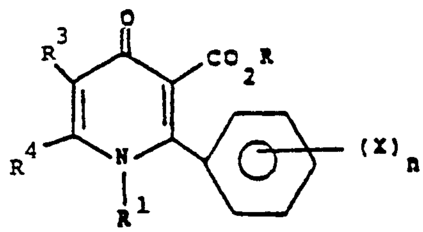

(IX)

This reaction is generally carried out in an inert solvent, such as toluene, xylene, benzene, chloroform, methylene chloride, methanol, ethanol or the like, at room temperature or at a temperature at which the water formed during the reaction can be removed by azeotropic distillation, using about 0 to 5% by weight of an acid catalyst such as p-toluenesulfonic acid, hydrochloric acid, sulfuric acid, methanesulfonic acid, or the like. The free acid, its salts, amides, and other esters can then be prepared by conventional techniques.

The reaction of 3-carboxy-4-pyrone ester of formula (VII) with excess amine of formula (VIII) in methanol or ethanol at 0—50°C also results in the formulation of a 2:1 amine:pyrone adduct of formula (X).

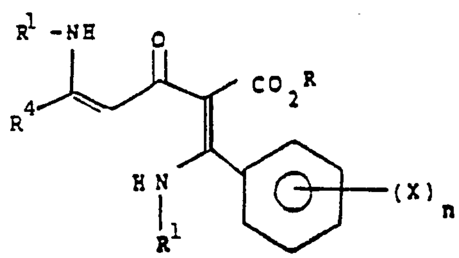

(X)

Compound (X) can be converted to (IX) by hydrolysis with dilute aqueous acids such as hydrochloric, sulfuric, trifluoroacetic or methanesulfonic at 0°—50°C. Alternately, 2:1 adduct (X) can be alkylated, halogenated or arylated with an alkyl halide, molecular chlorine or bromine or an activated aryl halide, respectively, in an inert solvent such as methylene chloride, benzene, tetrahydrofuran, diethyl ether and the like to provide a material of formula (XI) wherein $R^5$ is an alkyl group;

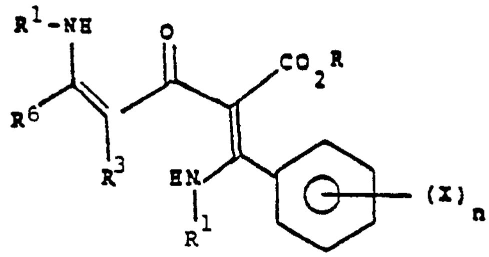

(XI)

This compound can be hydrolyzed in aqueous acids at 0—50°C as described above to yield the corresponding 1,5-dialkyl-2-aryl-4-oxonicotinate ester of formula (IX) where $R^3$ is an alkyl. The oxonicotinic acid esters produced in the above reactions can be converted to the free acids by hydrolysis with a strong base such as sodium hydroxide or potassium hydroxide and the like followed by neutralization with a strong acid.

Another route to the preparation of these compounds wherein $R^1$ and X are as defined above, $R^3$ is hydrogen and $R^4$ is methyl is depicted in the following reaction sequence:

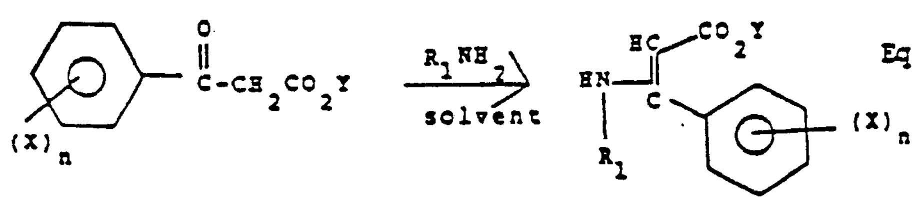


Eq. (1)

(XII) (XIII)

wherein Y is a ($C_1$—$C_6$) alkyl group.

(XIII) +

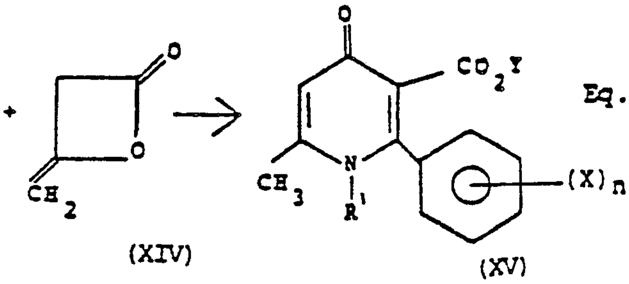

(XIV) (XV)

Eq. (2)

wherein Y is a ($C_1$—$C_6$) alkyl group.

Formula XV

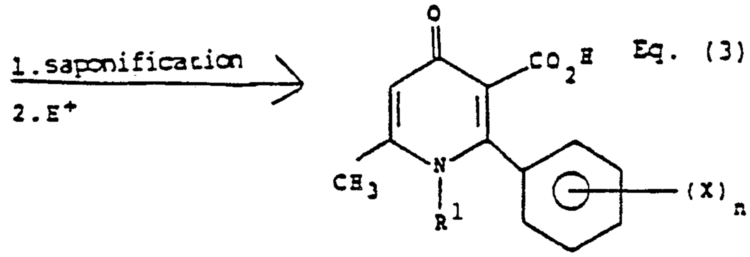


Eq. (3)

(XVI)

In the above reaction sequence the solvents for Eq. (1) can be selected from methanol, ethanol, water and the like and the reaction is run at temperatures from about 20° to about 100°C. In Eq. (2), the inert solvent is selected from ethers, methylene chloride, aromatic hydrocarbons, acetone, acetonitrile and the like and the reaction is run at temperatures from about 10° to about 150°C. In Eq. (3) the saponification reaction is run at 10°—100°C with a strong base, such as sodium or potassium hydroxide, and the alkali salt is converted to the free acid *via* mineral acids such as hydrochloride, sulfuric and the like.

Another route to the compounds wherein $R^1$ is defined above, $R^4$ is hydrogen or an alkyl group, and $R^3$ is hydrogen or alkyl is shown in equation (4) below.

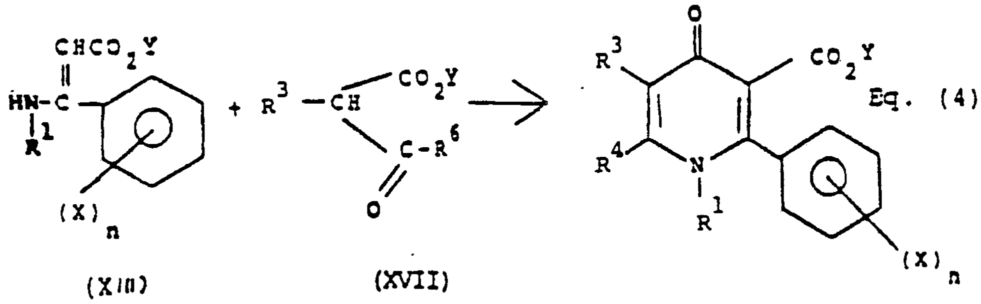

Eq. (4)

(XIII) (XVII)

wherein Y is an alkyl group.

In the reaction sequence of Eq. (4) the reaction can be run either neat or in an inert cosolvent optionally in the presence of an acid catalyst such as toluene sulfonic acid, sulfuric acid, acetic acid and the like at temperatures from about 100° to about 300°C.

A route to the 5-halo ($R^3$) compounds is shown in Eq. (5) below.

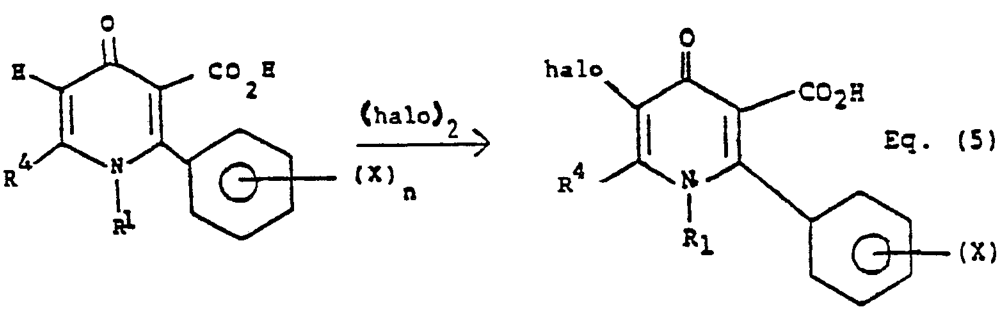


Eq. (5)

In this reaction sequence any protic solvent such as water, methanol, ethanol and the like can be utilized as the reaction medium and the reaction can be carried out at temperatures from about 10 to about 50°C.

(2) when $R^3$ is the phenyl group, $R^2$ is an alkyl group or independently the phenyl group and $R^4$ is a hydrogen atom or an alkyl group; the compounds can be prepared as follows:

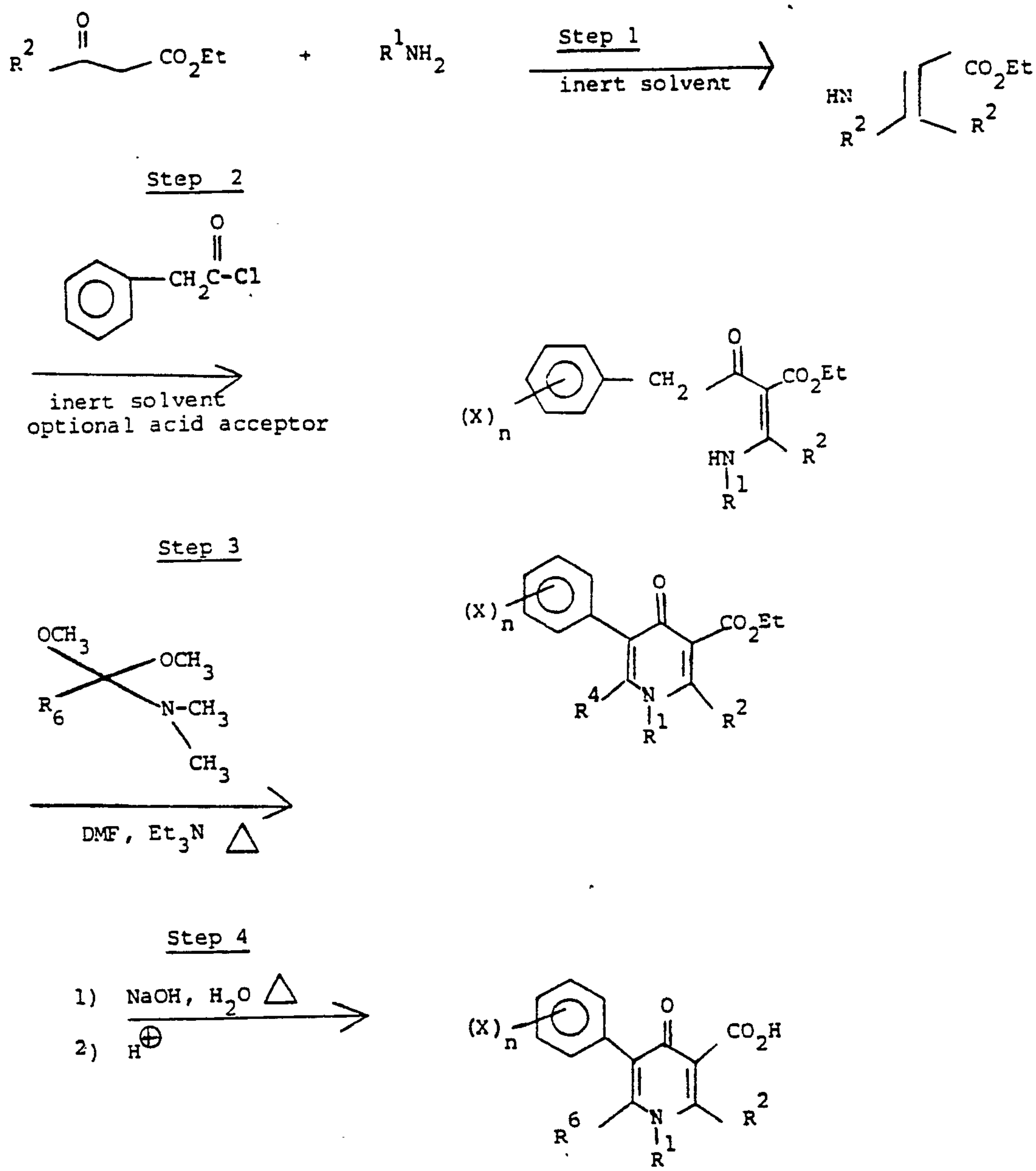


Step 1:
- (a) methanol, ethanol, water or similar inert solvents
- (b) 20° to 100°C

Step 2:
- (a) MDC, benzene, ether or other inert solvents
- (b) acid acceptor optional
- (c) 0°—100°, preferably 0—50°C

Step 3:
- (a) DMF solvent, excess amide acetal
- (b) basic catalyst optional ($Et_3N$)
- (c) 20—150°, preferably 50—100°C

Step 4:
- (a) aqueous NaOH, $Na_2CO_3$, other bases
- (b) optional miscible co-solvent (EtOH, MeOH)
- (c) acidification with HCl, $H_2SO_4$ etc.

(3) when $R^4$ is the phenyl group, $R^2$ is an optionally substituted ($C_2$—$C_6$)alkyl group or ($C_3$—$C_6$)alkenyl group and $R^3$ is a hydrogen atom, a ($C_1$—$C_6$)alkyl group or a halogen atom.

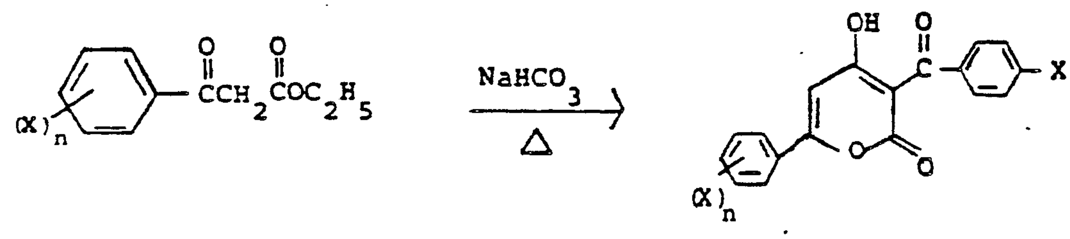


(XIX) (XX)

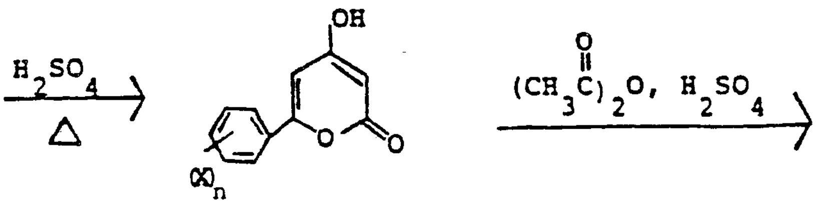


(XXI)

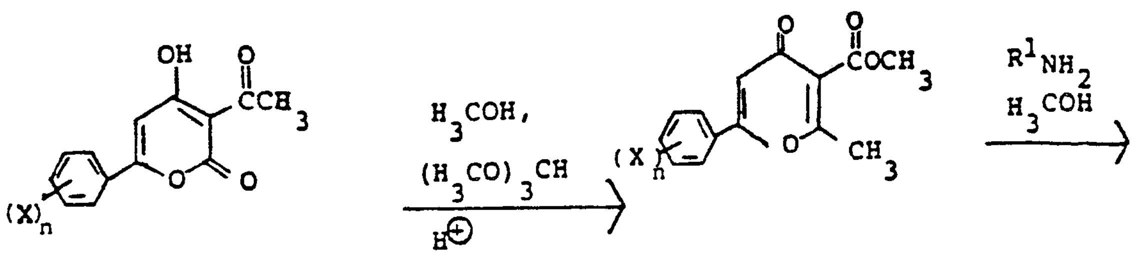


(XXII) (XXIII)

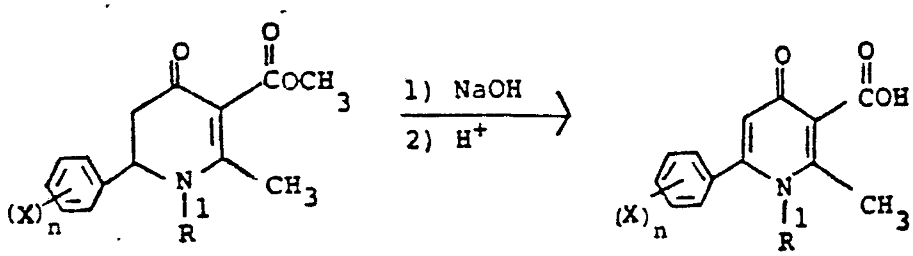


(XXIV) (XXV)

Alternatively, these compounds can be prepared by the following reaction sequence:

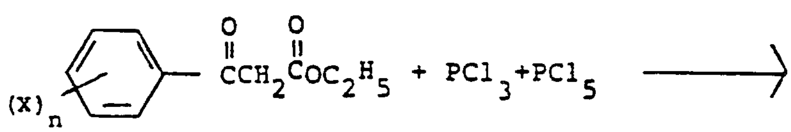


(XIX)

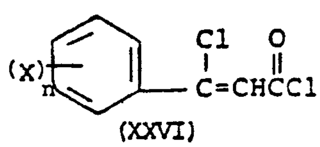

(XXVI)

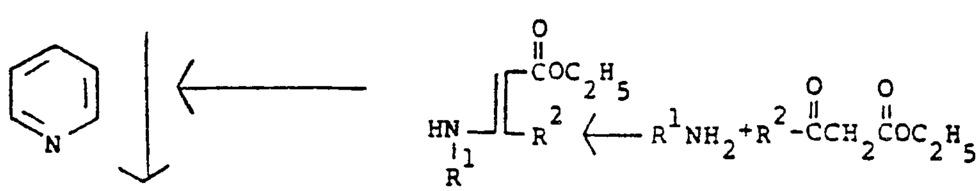

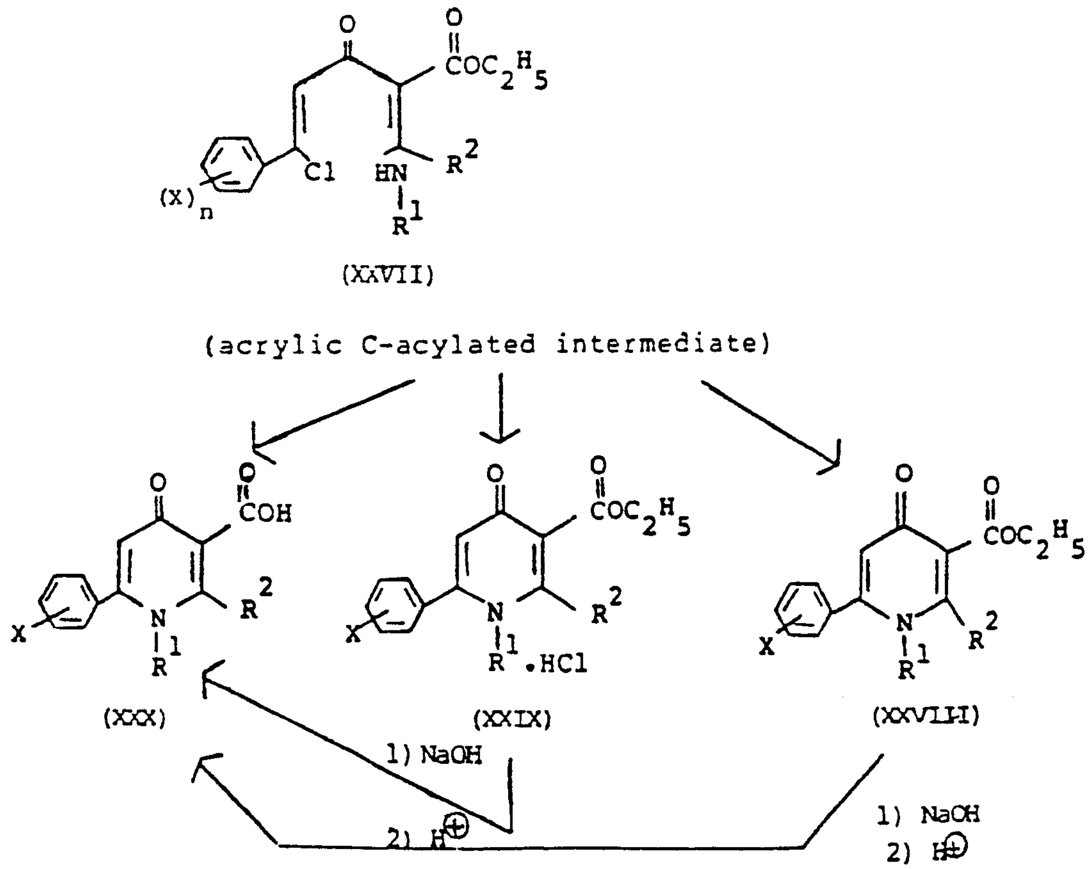


(XXVII)

(acrylic C-acylated intermediate)

(XXX) (XXIX) (XXVIII)

In the latter reaction sequence, the cyclization step yielded one or more of the pyridones, XXVIII, XXIX and XXX. In general, a pyrolysis (neat) of the acrylic C-acrylated intermediate (XXVII) yielded the oxonicotinic acid (XXX) directly. Cyclization of the acrylic intermediate (XXVII) in aprotic solvents at lower temperatures gave varied results depending on reaction conditions and the X, $R^1$ and $R^2$ substituent effects. In some cases, mixtures of products were obtained (ester, ester hydrochloride and acid), in other cases only one of the products was isolated.

(4) when $R^2$ and $R^4$ are each independently the phenyl group and $R^3$ is a hydrogen atom, an alkyl group or a halogen atom, the compounds can be prepared as follows:

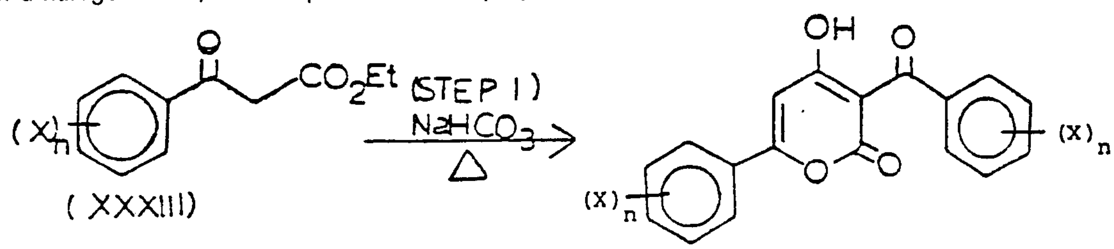


( XXXIII)

(XXXIV)

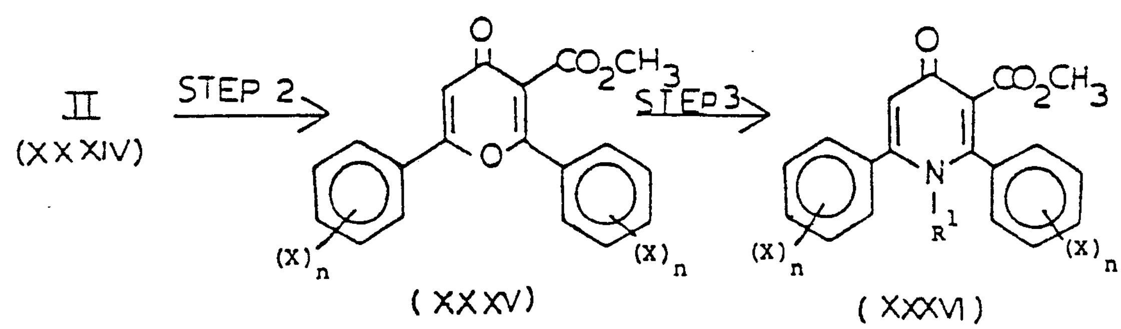


II (XXXIV)

( XXXV) ( XXXVI )

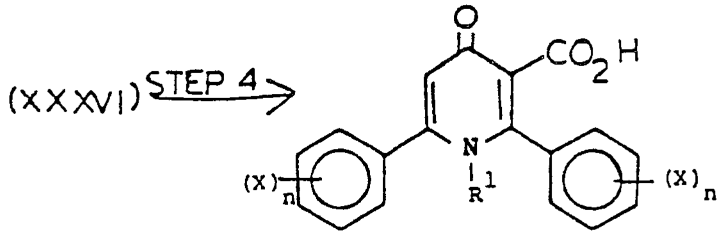


(XXXVII)

Step 1:
(a) $NaHCO_3$, $Na_2CO_3$;
(b) chlorobenzene, dichlorobenzene, toluene, decalin, other insert solvents;
(c) temp.: 50—250°C, preferably 100—200°C and
(d) similar to procedure described F. Arndt, B. Eistert, H. Schole and F. Aron, *Ber., 69*, 2373 (1936).

Step 2:
(a) methanol;
(b) trimethyl orthoformate dessicant;
(c) $H_2SO_4$, HCl, other strong acids;
(d) temp.: 25—200°C, preferably 50—150°C.

Step 3:
(a) alkyl amine;
(b) inert solvent, alcohols, ethers, hydrocarbons, water;
(c) acid catalyst optional;
(d) temp.: 0—150°C, preferably 25—50°C.

Step 4:
(a) aqueous base;
(b) optional inert cosolvent, methanol, DMSO;
(c) temp.: 20—150°C, preferably 50—100°C;
(d) any acid to neutralize.

Another route to these compounds of group (4) is:

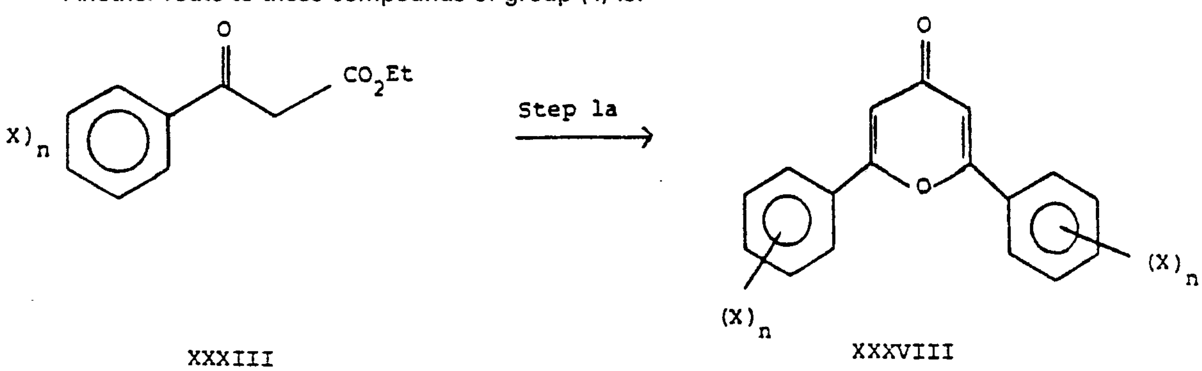


XXXIII XXXVIII

XXXVIII

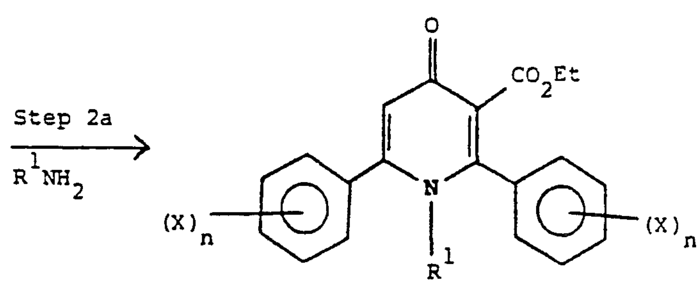


XXXIX

XXXIX

Step 3a

XXXVII

Step 1a:
(a) 1:10 of $P_2O_5$:$CH_3SO_3H$ by weight;
(b) temperature: 20—150°C, preferably 20—60°C.

Step 2a:
(a) alkyl amine;
(b) inert solvent, alcohols, ethers, hydrocarbons, water;
(c) acid catalyst optional;
(d) temperature: 0—150°C, preferably 25—50°C.

Step 3a:
(a) aqueous base;
(b) optional inert cosolvent, methanol, DMSO;
(c) temperature: 20—150°C, preferably 50—100°C;
(d) any acid to neutralize.

Still another route to these compounds of group (4) is shown by the following reaction sequence:

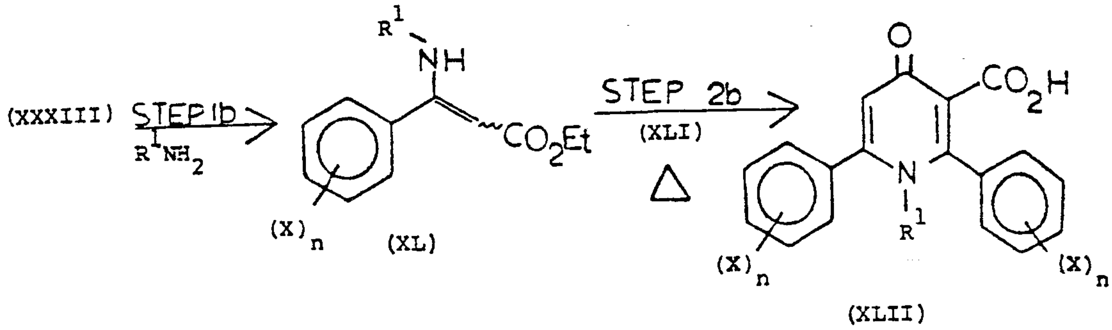

(XXXIII) (XL) (XLII)

Step 1b:
(a) $R^1NH_2$;
(b) inert solvent, methanol, $H_2O$;
(c) acid catalyst optional;
(d) temp.: 20—150°C, preferably 20—100°C.

Step 2b:
(a) run in inert solvent (methylene chloride, benzene, toluene, ether);
(b) acid acceptor optional (pyridine, $Et_3N$);
(c) temp.: 0°—150°C, preferably 10—50°C;
(d) residue heated 100—200°C, preferably 150—180°C.

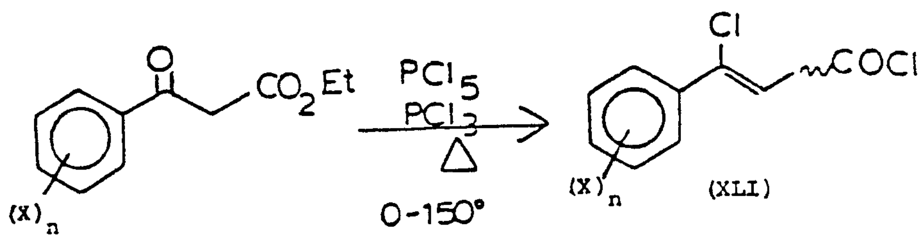

(XLI)

Yet another route to these halogenated compounds of group (4) is shown by the following reaction equation:

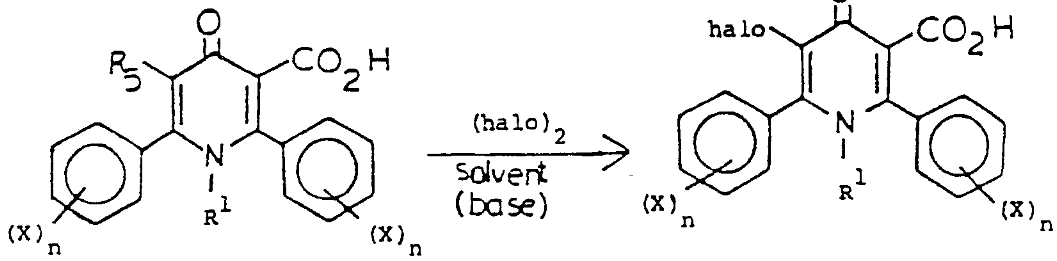


(a) carried out in compatible solvent (MeOH, water)
(b) room temperature

(c) optional addition of base (NaOH, $NA_2CO_3$, etc.)

The 4-oxo or thio-pyridazine apomixic agents can be prepared, for example, by the following routes:

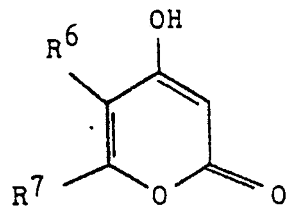

(1)

wherein $R^6$ is a hydrogen atom, an aralkyl group or an alkyl group and $R^7$ is as defined above, or a salt of a pyrone of formula (1), prepared by treating the pyrone with an equivalent of a suitable aqueous base such as potassium or sodium hydroxide, acetate, or carbonate, is reacted at about −10° to about 50°C in a polar solvent, such as water, methanol, ethanol, glyme, dimethylformamide, or the like, with a diazonium salt, such as diazonium chloride, prepared by conventional diazotization techniques from an amine of the formula

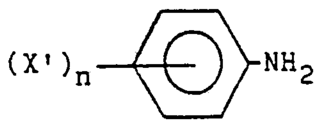

(2)

The product hydrazone, which has the formula

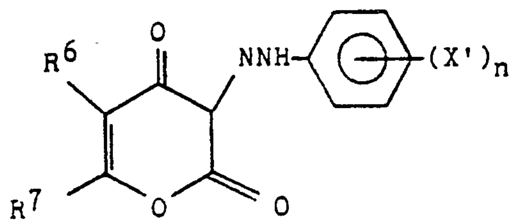

(3)

wherein $R^6$ and $R^7$ are as defined above, is then treated with either an aqueous acid, such as hydrochloric acid, trifluoroacetic acid, sulfuric acid, methanesulfonic acid, nitric acid, or the like, or an aqueous base, such as sodium carbonate, sodium hydroxide, or an alcoholic secondary amine such as morpholine, piperidine, dialkyl amine and the like, at a temperature of about 0° to about 150°C, preferably about 40° to about 100°C, to yield, by a rearrangement, a pyridazinone of the formula

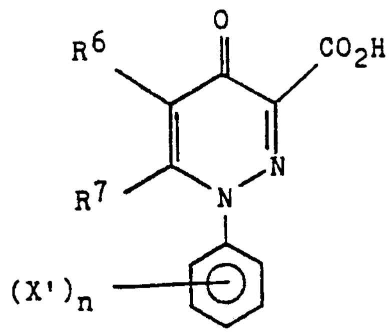

(4)

or salt thereof wherein $R^6$ and $R^7$ are as defined above.

The pryridazinones of the invention in which $R^6$ is a halogen atom can be prepared by reacting the corresponding pyridazinones in which $R^6$ is a hydrogen atom with one equivalent of a halogenating agent such as bromine, chlorine, sulfuryl bromide, sulfuryl chloride, or the like in a suitable inert solvent such as hexane, benzene, ethylene dichloride, methanol, or the like, at a temperature of about 0° to 50°C, preferably at room temperature.

(2) When $R^6$ is

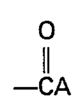

and A is hydrogen, alkyl or aralkyl and the remainder of the substituents are as defined above, the following synthesis route can be used:

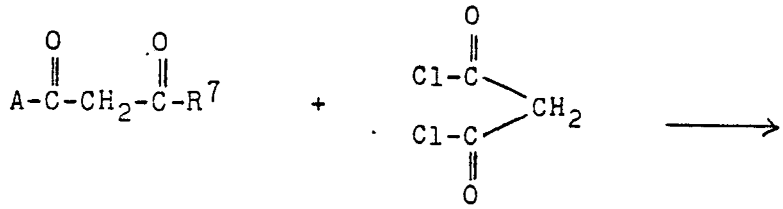

(5) (6)

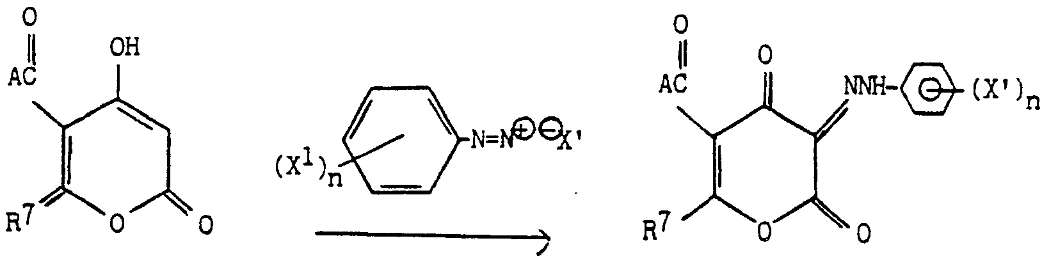

(7) (8) (9)

(9) 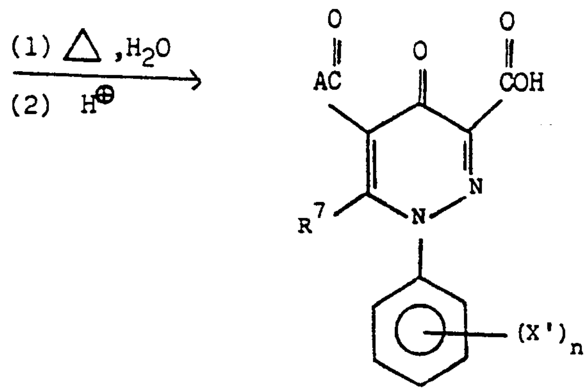


(10)

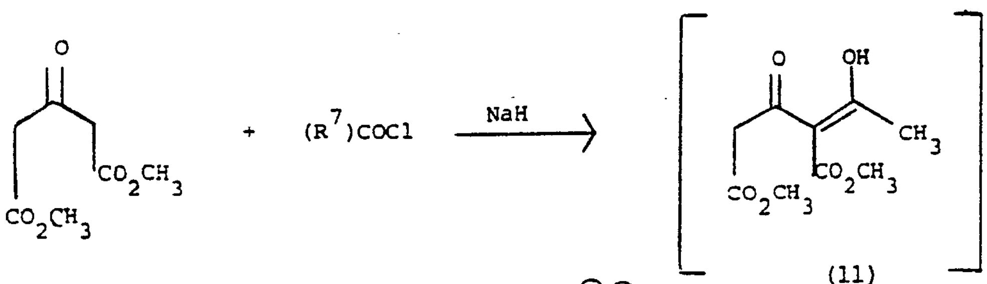


(11)

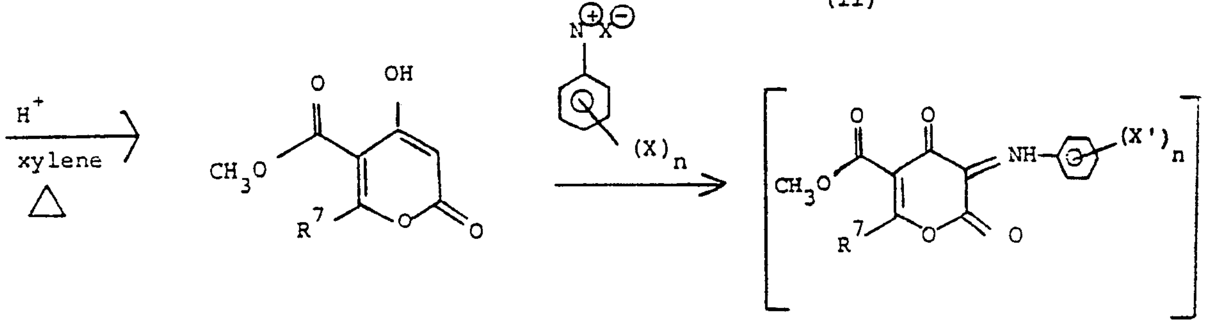


(12) (13)

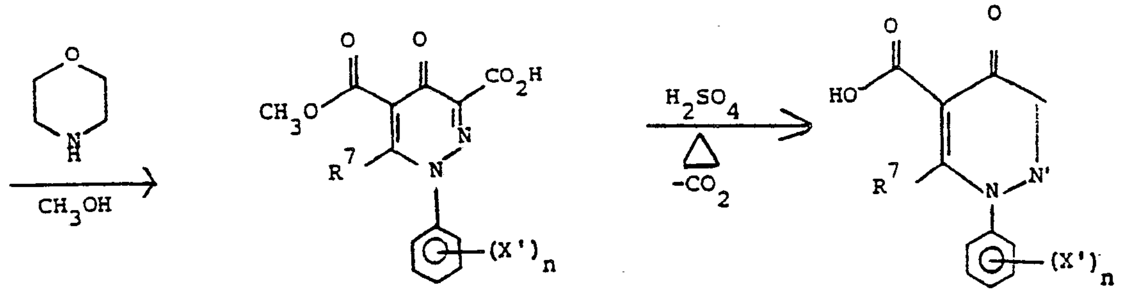


(14) (15)

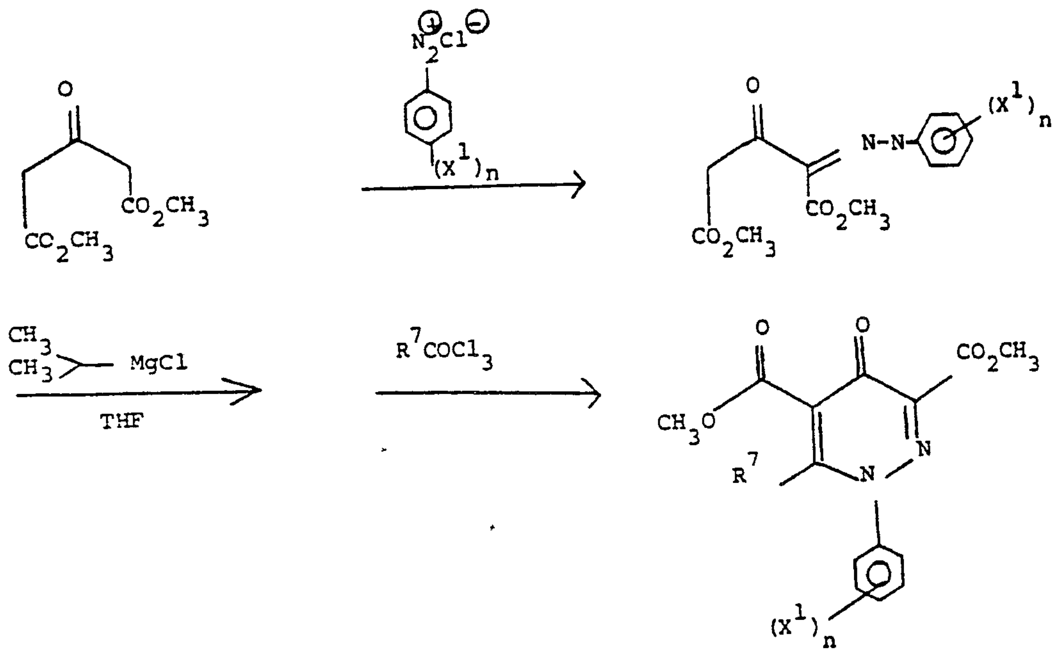


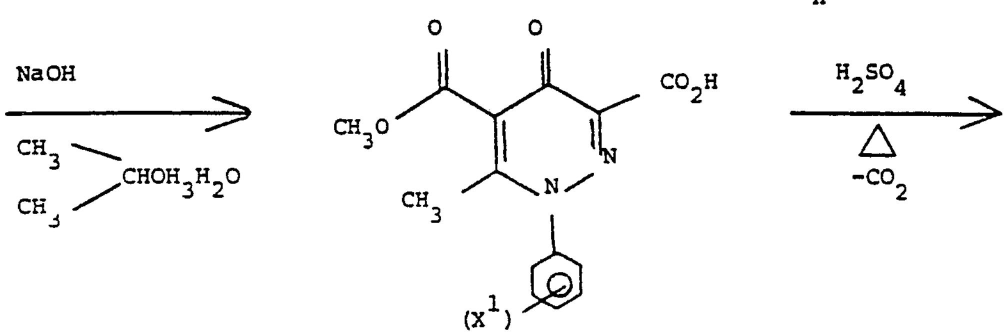


(15)

(14)

Salts of the pyridazinones of formulas 4, 10, 14 and 15, and their 5-halo analogues can be prepared by conventional techniques, such as by neutralization with an appropriate inorganic or organic base, in a solvent such as water or methanol.

Esters of the pyridazinones of formulas 4, 10, 14 and 15 are prepared by esterification with a suitable alcohol, preferably a $(C_1—C_{12})$alkanol. One convenient technique is a Fischer esterification, using anhydrous hydrochloric acid or sulfuric acid as a catalyst and the alcohol as the solvent. This esterification is generally carried out at about 35° to about 150°C, optionally using an inert cosolvent such as methylene chloride, ethylene chloride, diethyl ether, toluene, xylene, or the like.

Another convenient esterification technique is the reaction of the pyridazinone acylhalide formed as above with an appropriate alcohol utilizing the alcohol or any inert cosolvent for the reaction media. Alternatively a thionyl halide can be utilized to first form a halosulfinite with the appropriate alcohol and this halosulfinite can then be reacted with the pyridazinone carboxylic acid to form the appropriate ester.

These esterification procedures can be carried out at about 0° to about 80°C. The alcohol of the ester can also be used as the solvent in this reaction.

Yet another esterification technique involves the use of a halo carboxylate to form a pyridazinone acylanhydride followed by either thermal decomposition to eliminate carbon dioxide and form the desired ester or addition of an equimolar (or excess) amount of the alcohol of the ester, to form the desired ester. This reaction can initially be run at about 0°C and the temperature increased until the evoluation of carbon dioxide has ceased. This reaction is carried out in the presence of an acid scavenger in an inert solvent. The thioesters of the invention can be prepared as above by the replacement of a thioalcohol.

The pyridazinone carbamides of the invention can be prepared by standard synthetic routes such as the reaction of the acylhalide discussed above with an appropriate amine in an inert solvent at about 0° to about 150°C. Another convenient amination route utilizes the reaction of the pyridazinone carboxylic acid with an appropriate halocarboxylate to first form an acylanhydride which in turn is reacted with an appropriate amine at temperatures at about 25° to about 110°C in an inert solvent again with the evolution of carbon dioxide to form the appropriate carbamide.

The oxo-pyridazines and oxo-nicotinates are readily converted to their thio analogs using conventional techniques.

**Claims**

1. A process for producing haploid seed by inducing haploids in an angiosperm comprising applying an apomixic agent to a plant at a time which is between the occurrence of meiosis and anthesis in an amount sufficient to cause apomixis, allowing the plant to produce seed and collecting from the seed, viable, haploid seed.

2. A process as claimed in claim 1 for producing doubled haploids in an angiosperm which comprises the further steps of growing the haploid seed in an environment which fosters the development of the embryo into a plant which is capable of producing doubled haploid seed.

3. A process as claimed in claim 1 for producing genetically stable haploid seed, source of an homozygous plant line which comprises the further steps of growing the haploid seed in an environment which fosters the development of its embryo into a mature, seed producing plant wherein each seed is genetically stable and represents the source of an homozygous line.

4. The process of claim 3 wherein the homozygous plant lines are a cereal grain or forage crop, the apomixic agent is applied in an amount of from 0.03 to 11 kilograms per hectare and is applied to the plant at a time from the occurrence of meiosis to anthesis.

5. A process as claimed in claim 3 or 4 which comprises the further step of growing an homozygous plant line from the seed produced by the doubled haploid plants.

6. The process of claim 1, 2, or 3 wherein the apomixic agent is applied in an amount of 0.01 to 22 kilograms per hectare.

7. The process of claim 6 wherein the apomixic agent is applied to a monocotyledonous plant at a time which is between meiosis and anthesis.

8. The process of claim 6 wherein the apomixic agent comprises one or more of the following: 1 - (4 - chloro-phenyl) - 1,4 - dihydro - 4 - oxo - 6 - methylpyridazine - 3 - carboxylic acid, 1 - (4 - chloro-phenyl) - 1,4 - dihydro - 4 - oxo - 5 - carboxy-methyl - 6 - ethylpyridazine - 3 - carboxylic acid, 1 - (4 - chloro-phenyl) - 1,4 - dihydro - 4 - oxo - 5 - propionyl - 6 - methylpyridazine - 3 - carboxylic acid, 1 - ethyl - 6 - methyl - 2 - (4 - chlorophenyl) - 4 - oxonicotinic acid, 1 - ethyl - 6 - methyl - 2 - (3,4 - dichloro-phenyl) - 4 - oxonicotinic acid, 1 - (3,4 - dichloro-phenyl) - 1,4 - dihydro - 4 - oxo - 5 - carboxy-methyl - 6 - methylpyridazine and 1 - phenyl - 1,4 - dihydro - 4 - oxo - 5 - carboxymethyl - 6 - ethylpyridazine and water soluble salts thereof and methyl or ethyl 1 - (4 - chlorophenyl) - 1,4 - dihydro - 4 - oxo - 6 - ethylpyridazine - 5 - carboxylate and methyl or ethyl 1 - (4 - bromophenyl) - 1,4 - dihydro - 4 - oxo - 6 - ethylpyridazine - 5 - carboxylate.

9. The process of claim 8 wherein the apomixic agent is applied in an amount of from 0.03 to 11 kilograms per hectare.

10. A process for inducing apomixis in an angiosperm plant comprising applying to the plant at a time from flower initiation to anthesis and in an amount sufficient to cause apomixis, an apomixic agent which comprises one or more of those 4-oxo(thio)nicotinates and 4-oxo(thio)pyridazines which have growth regulant properties.

11. The process of claim 10 wherein the apomixic agent is an effective chemical hybridizing agent in relation to the plant to which it is applied.

12. A process as claimed in claim 1 or 2 wherein the plant comprises a cereal grain or forage crop plant and the apomixic agent is applied at a time from meiosis until just before anthesis and in an amount of from 0.03 to 11 kilograms per hectare.

13. A process as claimed in claim 2 wherein the plant comprises a cereal grain or forage crop plant and the apomixic agent is applied at a time from meiosis until just before anthesis in an amount of from 0.03 to 11 kilograms per hectare.

14. The process of claim 10 or 13 wherein the apomixic agent comprises one or more 4-oxo(thio)nicotinates or 4-oxo(thio)pyridazines of the following structures:

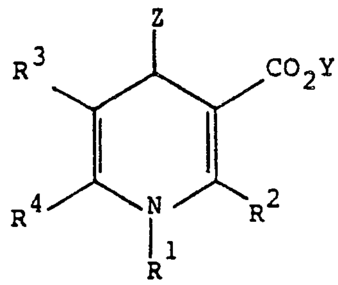

wherein

Z is an oxygen or sulfur atom;

$R^1$ is an optionally substituted ($C_1$—$C_6$)alkyl or ($C_2$—$C_6$)alkenyl; at least one of $R^2$, $R^3$ or $R^4$ is a

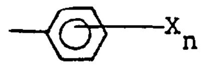

("the phenyl group") wherein

when $R^2$ is "the phenyl group", $R^3$ is a hydrogen atom, an alkyl group or a halogen atom and $R^4$ is a hydrogen atom, an alkyl group or independently the phenyl group;

when $R^3$ is "the phenyl group", $R^2$ is an alkyl group or independently "the phenyl group", and $R^4$ is a hydrogen atom or an alkyl group;

when $R^4$ is "the phenyl group", $R^2$ is an optionally substituted ($C_2$—$C_6$)alkyl group, ($C_3$—$C_6$)alkenyl group or independently "the phenyl group" and $R^3$ is a hydrogen atom, a ($C_1$—$C_6$)alkyl group or a halogen atom; and

Y is a hydrogen atom, an agronomically acceptable salt thereof or an alkyl group;

X is a hydrogen atom, a halogen atom, a trihalomethyl group, a ($C_1$—$C_6$)alkyl group, a nitro group, a cyano group or a ($C_1$—$C_6$)alkoxy group; and

n is an integer of from 1 to 3; and

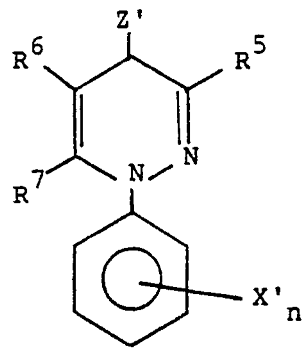

wherein

Z' is an oxygen or sulfur atom;

$R^5$ is a hydrogen atom, halogen atom,

$$-\overset{\displaystyle O}{\overset{\|}{C}}NRR$$

wherein R and R are each independently hydrogen, alkyl, alkyl substituted with a carbonyl or carboxy group,

$$-\overset{\displaystyle O}{\overset{\|}{C}}WR^8$$

wherein W is an oxygen or sulfur atom and $R^8$ is a hydrogen atom, alkyl group, aryloxy, alkoxyalkyl, alkylhalogen, alkylhydroxy, iminoalkyl, aryl, alkenyl, cyclo($C_3$—$C_6$)alkyl or cycloalkyl alkyl group;

$R^6$ is a hydrogen atom, alkyl,

$$-\overset{\displaystyle O}{\overset{\|}{C}}A$$

wherein A is hydrogen, alkyl, aralkyl, hydroxyl, alkoxy, thio, alkylthio or $-NR^9R^{10}$ wherein $R^9$ and $R^{10}$ are

each independently hydrogen, alkyl, alkyl substituted with a carbonyl or carboxy group, provided that $R^5$ and $R^6$ are not both hydrogen atoms; and when $R^5$ is a hydrogen or halogen atom, then $R^6$ is

$$-\overset{\overset{\displaystyle O}{\|}}{C}A'$$

wherein A′ is an alkoxy, thioalkyl or $—NR^9R^{10}$ group; and

$R^7$ is alkyl, aryl or aryl substituted with up to three substituents selected from the group consisting of halogen, nitro, trihalomethyl, $(C_1—C_4)$alkoxy, $(C_1—C_4)$alkyl or cyano;

n is 0, 1, 2 or 3;

each X′ is independently a hydrogen atom, halogen atom, trihalomethyl, alkyl, alkoxy, cyano or nitro group; and the agronomically acceptable salts thereof.

15. The process of claim 14 wherein:

Z and Z′ are oxygen atoms;

X and X′ are each independently a hydrogen, a halogen or trifluoromethyl group;

n is 1 or 2;

$R^1$ is a $(C_1—C_4)$alkyl group;

$R^2$ or $R^4$ is "the phenyl group";

when $R^2$ is "the phenyl group", $R^3$ is a $(C_1—C_3)$alkyl, bromine or a hydrogen atom; $R^4$ is a hydrogen atom, a halogen atom or a $(C_1—C_4)$alkyl group;

when $R^4$ is "the phenyl group", $R^2$ is a hydrogen atom, a halogen atom or a $(C_1—C_4)$alkyl group; $R^3$ is a halogen atom or a hydrogen atom;

Y is a hydrogen atom, $(C_1—C_6)$alkyl or an alkali metal or alkaline earth metal cation;

$R^5$ is hydrogen, halogen or

$$-\overset{\overset{\displaystyle O}{\|}}{C}WR^8$$

wherein W is an oxygen atom and $R^8$ is a hydrogen atom, $(C_1—C_4)$alkyl, $(C_1—C_4)$alkylhalogen, $(C_1—C_4)$alkylhydroxy, $(C_1—C_4)$-alkoxy$(C_1—C_4)$alkyl, $(C_1—C_2)$phenyl, $(C_1—C_4)$alkenyl or imino$(C_1—C_4)$alkyl group or W is sulfur and $R^5$ is a $(C_1—C_4)$alkyl group;

$R^6$ is hydrogen, $(C_1—C_4)$alkyl,

$$-\overset{\overset{\displaystyle O}{\|}}{C}A$$

wherein A is $(C_1—C_4)$alkyl, $(C_1—C_4)$alkoxy, $(C_1—C_4)$alkylthio or $—NR^9R^{10}$ and when $R^5$ is hydrogen or halogen, then $R^6$ is

$$-\overset{\overset{\displaystyle O}{\|}}{C}A$$

and A is $(C_1—C_4)$alkoxy or $(C_1—C_4)$alkylthio;

$R^7$ is a $(C_1—C_4)$alkyl group; and the agronomically acceptable salts thereof.

16. The process of claim 15 wherein:

Z and Z′ are oxygen atoms;

X is a hydrogen, chloro, bromo, fluoro or iodo atom;

X′ is a chloro, fluoro or iodo atom;

n is 1 or 2;

$R^1$ is a methyl, ethyl or *n*-propyl group;

$R^2$ is a $(C_1—C_4)$alkyl group

$R^3$ is a hydrogen atom;

$R^4$ is "the phenyl group";

Y is a sodium or potassium cation;

$R^5$ is

$$-\overset{\overset{\displaystyle O}{\|}}{C}WR^8,$$

wherein W is an oxygen atom and $R^8$ is a hydrogen atom or a sodium, potassium or triethanolamine salt thereof, or a $(C_1—C_2)$alkyl group;

$R^6$ is a hydrogen atom or

$$-\overset{\overset{\displaystyle O}{\|}}{C}A'$$

wherein A' is hydroxy or $(C_1-C_2)$alkoxy group;

$R^7$ is a methyl, ethyl or *n*-propyl group; and the agronomically acceptable salts thereof.

17. The process of claim 12 or 13 wherein the crop is wheat, barley, oats, rice, rye, maize, sorghum, millets or triticale, the apomixic agent is applied in an amount of 0.1 to 6 kilograms per hectare and the apomixic agent comprises one or more of the compounds: 1 - (4 - chlorophenyl) - 1,4 - dihydro - 4 - oxo - 6 - methylpyridazine - 3 - carboxylic acid, 1 - (4 - chlorophenyl) - 1,4 - dihydro - 4 - oxo - 5 - carboxymethyl - 6 - methylpyridazine - 3 - carboxylic acid, 1 - (4 - chlorophenyl) - 1,4 - dihydro - 4 - oxo - 5 - propionyl - 6 - methyl-pyridazine - 3 - carboxylic acid, 1 - ethyl - 6 - methyl - 2 - (4 - chlorophenyl) - 4 - oxonicotinic acid, 1 - ethyl - 6 - methyl - 2 - (3,4 - dichloro-phenyl) - 4 - oxonicotinic acid, 1 - (3,4 - dichlorophenyl) - 1,4 - dihydro - 4 - oxo - 5 - carboxymethyl - 6 - methyl-pyridazine and 1 - phenyl - 1,4 - dihydro - 4 - oxo - 5 - carboxymethyl - 6 - ethylpyridazine and water soluble salts thereof and methyl or ethyl 1 - (4 - chlorophenyl) - 1,4 - dihydro - 4 - oxo - 6 - ethylpyridazine - 5 - carboxylate and methyl or ethyl 1 - (4 - bromophenyl) - 1,4 - dihydro - 4 - oxo - 6 - ethylpyridazine - 5 - carboxylate.

**Patentansprüche**

1. Verfahren zur Herstellung von haploidem Samen durch Induzieren von Haplonten in einem Angiosperm, umfassend die Anwendung eines Apomixis-Mittels auf eine Pflanze zu einem Zeitpunkt zwischen dem Auftreten der Meiosis und der Blüte, in einer ausreichenden Menge, um die Apomixis zu bewirken, Produzierenlassen von Samen durch die Pflanze und Sammeln von lebensfähigem haploidem Samen aus den Samen.

2. Verfahren nach Anspruch 1 zur Herstellung von verdoppelten Haplonten in einem Angiosperm, das die weiteren Stufen des Wachstums des haploiden Samens in einer Umgebung umfaßt, die die Entwicklung des Embryos zu einer Pflanze fördert, die geeignet ist, verdoppelte haploide Samen zu erzeugen.

3. Verfahren nach Anspruch 1 zur Herstellung von genetisch stabilem haploidem Samen, als Quelle einer homozygoten Pflanzenlinie, das die weiteren Stufen des Wachstums des haploiden Samens in einer Umgebung umfaßt, die die Entwicklung seines Embryos zu einer reifen, samenproduzierenden Pflanze fördert, worin jeder Samen genetisch stabil ist und die Quelle einer homozygoten Linie darstellt.

4. Verfahren nach Anspruch 3, bei dem die homozygoten Pflanzenlinien ein Korn-Getreide oder eine Futterfrucht sind, das Apomixis-Mittel in einer Menge von 0,03 bis 11 Kilogramm pro Hektar angewendet wird und auf die Pflanze zu einem Zeitpunkt von Auftreten der Meiosis bis zur Blüte angewendet wird.

5. Verfahren nach Anspruch 3 oder 4, das die weitere Stufe des Wachstums einer homozygoten Pflanzenlinie aus dem durch die verdoppelten haploiden Pflanzen erzeugten Samen umfaßt.

6. Verfahren nach Anspruch 1, 2 oder 3, bei dem das Apomixis-Mittel in einer Menge von 0,01 bis 22 Kilogramm pro Hektar angewendet wird.

7. Verfahren nach Anspruch 6, bei dem das Apomixis-Mittel auf eine Monocotyledonen-Pflanze zu einem Zeitpunkt, der zwischen der Meiosis und der Blüte liegt, angewendet wird.

8. Verfahren nach Anspruch 6, bei dem das Apomixis-Mittel eines oder mehrere der folgenden umfaßt: 1 - (4 - Chlorphenyl) - 1,4 - dihydro - 4 - oxo - 6 - methylpyridazin - 3 - carbonsäure, 1 - (4 - Chlorphenyl) - 1,4 - dihydro - 4 - oxo - 5 - carboxy-methyl - 6 - ethylpyridazin - 3 - carbonsäure, 1 - (4 - Chlorphenyl) - 1,4 - dihydro - 4 - oxo - 5 - propionyl - 6 - methylpyridazin - 3 - carbonsäure, 1 - Ethyl - 6 - methyl - 2 - (4 - chlorphenyl) - 4 - oxonicotinsäure, 1 - Ethyl - 6 - methyl - 2 - (3,4 - dichlorphenyl) - 4 - oxonicotinsäure, 1 - (3,4 - Dichlorphenyl) - 1,4 - dihydro - 4 - oxo - 5 - carboxymethyl - 6 - methylpyridazin und 1 - Phenyl - 1,4 - dihydro - 4 - oxo - 5 - carboxymethyl - 6 - ethylpyridazin und wasserlösliche Salze davon und Methyl- oder Ethyl - 1 - (4 - chlorphenyl) - 1,4 - dihydro - 4 - oxo - 6 - ethylpyridazin - 5 - carboxylat und Methyl- oder Ethyl - 1 - (4 - bromphenyl) - 1,4 - dihydro - 4 - oxo - 6 - ethylpyridazin - 5 - carboxylat.

9. Verfahren nach Anspruch 8, bei dem das Apomixis-Mittel in einer Menge von 0,03 bis 11 Kilogramm pro Hektar angewendet wird.

10. Verfahren zur Induzierung von Apomixis in einer Angiosperm-Pflanze, umfassend die Anwendung auf die Pflanze zu einem Zeitpunkt von der Blüten-Initiierung bis zur Blüte und in einer ausreichenden Menge zur Bewirkung der Apomixis, von einem Apomixis-Mittel, das eines oder mehrere umfaßt von 4-Oxo(thio)nicotinaten und 4-Oxo(thio)pyridazinen, die wachstumsregulierende Eigenschaften haben.

11. Verfahren nach Anspruch 10, bei dem das Apomixis-Mittel ein wirksames chemisches Hybridisierungsmittel, bezogen auf die Pflanze auf die es angewendet wird, ist.

12. Verfahren nach Anspruch 1 oder 2, bei dem die Pflanze ein Korn-Getreide oder eine Futterfrucht-Pflanze umfaßt und das Apomixis-Mittel zu einem Zeitpunkt von der Meiosis bis unmittelbar vor der Blüte und in einer Menge von 0,03 bis 11 Kilogramm pro Hektar angewendet wird.

13. Verfahren nach Anspruch 2, bei dem die Pflanze eine Korn-Getreide- oder eine Futterfrucht-Pflanze umfaßt und das Apomixis-Mittel zu einem Zeitpunkt von der Meiosis bis unmittelbar vor der Blüte in einer Menge von 0,03 bis 11 Kilogramm pro Hektar angewendet wird.

14. Verfahren nach Anspruch 10 oder 13, bei dem das Apomixis-Mittel eines oder mehrere 4-Oxo(thio)nicotinate oder 4-Oxo(thio)pyridazine der folgenden Strukturen umfaßt:

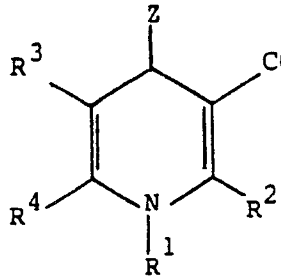

worin

Z ein Sauerstoff- oder Schwefelatom darstellt;

$R^1$ ein gegebenenfalls substituiertes $(C_1—C_6)$Alkyl oder $(C_2—C_6)$Alkenyl ist; wobei mindestens eines von $R^2$, $R^3$ oder $R^4$ ein

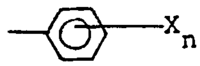

("die Phenylgruppe") ist, worin

wenn $R^2$ "die Phenylgruppe" ist, $R^3$ ein Wasserstoffatom, eine Alkylgruppe oder ein Halogenatom ist und $R^4$ ein Wasserstoffatom, eine Alkylgruppe oder unabhängig die Phenylgruppe ist;

wenn $R^3$ "die Phenylgruppe" ist, $R^2$ eine Alkylgruppe oder unabhängig "die Phenylgruppe" ist und $R^4$ ein Wasserstoffatom oder eine Alkylgruppe ist;

wenn $R^4$ "die Phenylgruppe" ist, $R^2$ eine gegebenenfalls substituierte $(C_2—C_6)$Alkylgruppe, $(C_3—C_6)$Alkenylgruppe oder unabhängig "die Phenylgruppe" ist und $R^3$ ein Wasserstoffatom, eine $(C_1—C_6)$Alkylgruppe oder ein Halogenatom ist; und

Y ein Wasserstoffatom, ein landwirtschaftlich brauchbares Salz davon oder eine Alkylgruppe ist;

X ein Wasserstoffatom, ein Halogenatom, eine Trihalogenmethylgruppe, eine $(C_1—C_6)$Alkylgruppe, eine Nitrogruppe, eine Cyanogruppe oder eine $(C_1—C_6)$Alkoxygruppe ist; und

n eine ganze Zahl von 1 bis 3 ist; und

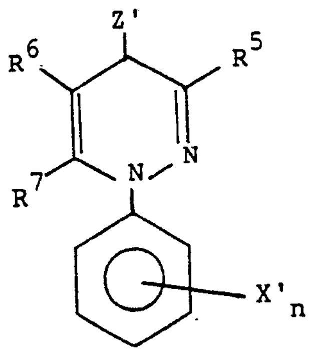

worin

Z' ein Sauerstoff- oder Schwefelatom ist;

$R^5$ die Bedeutung hat von einem Wasserstoffatom; Halogenatom,

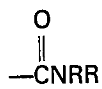

worin R und R jeweils unabhängig sind Wasserstoff, Alkyl, Alkyl substituiert mit einer Carbonyl- oder Carboxygruppe,

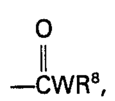

worin W ein Sauerstoff- oder Schwefelatom ist und $R^8$ bedeutet ein Wasserstoffatom, die Alkylgruppe, die

Aryloxy-, Alkoxyalkyl-, Alkylhalogen-, Alkylhydroxy-, Iminoalkyl-, Aryl-, Alkenyl-, Cyclo($C_3$—$C_6$)alkyl- oder Cycloalkyl-alkylgruppe;

$R^6$ bedeutet ein Wasserstoffatom, Alkyl,

$$-\overset{O}{\overset{\|}{C}}A,$$

worin A bedeutet Wasserstoff, Alkyl, Aralkyl, Hydroxyl, Alkoxy, Thio, Alkylthio oder —$NR^9R^{10}$, worin $R^9$ und $R^{10}$ jeweils unabhängig sind Wasserstoff, Alkyl, Alkyl substituiert mit einer Carbonyl- oder Carboxygruppe, vorausgesetzt, daß $R^5$ und $R^6$ nicht beide Wasserstoffatome sind; und wenn $R^5$ ein Wasserstoff- oder Halogenatom ist, dann ist $R^6$

$$-\overset{O}{\overset{\|}{C}}A',$$

worin A' eine Alkoxy-, Thioalkyl- oder —$NR^9R^{10}$ Gruppe ist; und

$R^7$ Alkyl, Aryl oder Aryl substituiert mit bis zu drei Substituenten ausgewählt aus der Gruppe bestehend aus Halogen, Nitro, Trihalogenmethyl, ($C_1$—$C_4$)Alkoxy, ($C_1$—$C_4$)Alkyl oder Cyano ist;

n die Bedeutung von 0, 1, 2 oder 3 hat;

jedes X' unabhängig ist ein Wasserstoffatom, Halogenatom, eine Trihalogenmethyl-, Alkyl-, Alkoxy-, Cyano- oder Nitrogruppe; und die landwirtschaftlich brauchbaren Salze davon.

15. Verfahren nach Anspruch 14, worin

Z und Z' Sauerstoffatome sind;

X und X' jeweils unabhängig ein Wasserstoff, ein Halogen oder eine Trifluormethylgruppe sind;

n die Bedeutung von 1 oder 2 hat;

$R^1$ eine ($C_1$—$C_4$)Alkylgruppe ist;

$R^2$ oder $R^4$ "die Phenylgruppe" ist;

wenn $R^2$ "die Phenylgruppe" ist, ist $R^3$ ein ($C_1$—$C_3$)Alkyl, Brom oder ein Wasserstoffatom; $R^4$ ist ein Wasserstoffatom, ein Halogenatom oder eine ($C_1$—$C_4$)Alkylgruppe;

wenn $R^4$ "die Phenylgruppe" ist, ist $R^2$ ein Wasserstoffatom, ein Halogenatom oder eine ($C_1$—$C_4$)Alkylgruppe; $R^3$ ist ein Halogenatom oder ein Wasserstoffatom;

Y ein Wasserstoffatom, ($C_1$—$C_6$)Alkyl oder ein Alkalimetall- oder Erdalkalimetall-Kation ist;

$R^5$ bedeutet Wasserstoff, Halogen oder

$$-\overset{O}{\overset{\|}{C}}WR^8,$$

worin W die Bedeutung hat von einem Sauerstoffatom und $R^8$ bedeutet ein Wasserstoffatom, ($C_1$—$C_4$)Alkyl, ($C_1$—$C_4$)Alkylhalogen, ($C_1$—$C_4$)Alkylhydroxy, ($C_1$—$C_4$)Alkoxy-($C_1$—$C_4$)alkyl, ($C_1$—$C_2$)Phenyl, ($C_1$—$C_4$)Alkenyl oder die Imino-($C_1$—$C_4$)alkylgruppe, oder W Schwefel ist und $R^5$ eine ($C_1$—$C_4$)Alkylgruppe ist;

$R^6$ die Bedeutung hat von Wasserstoff, ($C_1$—$C_4$)Alkyl,

$$-\overset{O}{\overset{\|}{C}}A,$$

worin A die Bedeutung hat von ($C_1$—$C_4$)Alkyl, ($C_1$—$C_4$)Alkoxy, ($C_1$—$C_4$)Alkylthio oder —$NR^9R^{10}$, und wenn $R^5$ Wasserstoff oder Halogen ist, dann ist $R^6$

$$-\overset{O}{\overset{\|}{C}}A$$

und A ist ($C_1$—$C_4$)Alkoxy oder ($C_1$—$C_4$)Alkylthio;

$R^7$ eine ($C_1$—$C_4$)Alkylgruppe ist; und die landwirtschaftich brauchbaren Salze davon.

16. Verfahren nach Anspruch 15, worin

Z und Z' Sauerstoffatome sind;

X ein Wasserstoff-, Chlor-, Brom-, Fluor- oder Jodatom ist;

X' ein Chlor-, Fluor- oder Jodatom ist;

n 1 oder 2 ist;

$R^1$ eine Methyl-, Ethyl- oder n-Propylgruppe ist;

$R^2$ eine ($C_1$—$C_4$)Alkylgruppe ist;

$R^3$ ein Wasserstoffatom ist;
$R^4$ "die Phenylgruppe" ist;
Y ein Natrium- oder Kalium-Kation ist;
$R^5$

$$\begin{matrix} O \\ \| \\ -CWR^8 \end{matrix}$$

ist, worin W ein Sauerstoffatom ist und $R^8$ ein Wasserstoffatom oder ein Natrium-, Kalium- oder Triethanolaminsalz davon oder eine $(C_1—C_2)$Alkylgruppe ist;
$R^6$ ein Wasserstoffatom oder

$$\begin{matrix} O \\ \| \\ -CA' \end{matrix}$$

ist, worin A' Hydroxy oder eine $(C_1—C_2)$Alkoxygruppe ist;
$R^7$ eine Methyl-, Ethyl- oder n-Propylgruppe ist; und die landwirtschaftlich brauchbaren Salze davon.

17. Verfahren nach Anspruch 12 oder 13, worin die Frucht Weizen, Gerste, Hafer, Reis, Roggen, Mais, Sorghum, Hirse oder Triticale ist, das Apomixis-Mittel in einer Menge von 0,1 bis 6 Kilogramm pro Hektar aufgetragen wird und das Apomixis-Mittel eine oder mehrere der Verbindungen umfaßt: 1 - (4 - Chlorphenyl) - 1,4 - dihydro - 4 - oxo - 6 - methylpyridazin - 3 - carbonsäure, 1 - (4 - Chlorphenyl) - 1,4 - dihydro - 4 - oxo - 5 - carboxymethyl - 6 - methylpyridazin - 3 - carbonsäure, 1 - (4 - Chlorphenyl) - 1,4 - dihydro - 4 - oxo - 5 - propionyl - 6 - methylpyridazin - 3 - carbonsäure, 1 - Ethyl - 6 - methyl - 2 - (4 - chlorphenyl) - 4 - oxonicotinsäure, 1 - Ethyl - 6 - methyl - 2 - (3,4 - dichlorphenyl) - 4 - oxonicotinsäure, 1 - (3,4 - Dichlorphenyl) - 1,4 - dihydro - 4 - oxo - 5 - carboxy-methyl - 6 - methylpyridazin und 1 - Phenyl - 1,4 - dihydro - 4 - oxo - 5 - carboxymethyl - 6 - ethylpyridazin und wasserlösliche Salze davon und Methyl- oder Ethyl - 1 - (4 - chlorphenyl) - 1,4 - dihydro - 4 - oxo - 6 - ethylpyridazin - 5 - carboxylat und Methyl- oder Ethyl - 1 - (4 - bromphenyl) - 1,4 - dihydro - 4 - oxo - 6 - ethylpyridazin - 5 - carboxylat.

**Revendications**

1. Procédé de production de semences haploïdes par induction d'haploïdes chez un agiosperme dans lequel on applique un agent apomixique à une plante à un moment qui se situe entre la survenue de la méïose et de l'anthèse en une quantité suffisante pour provoquer l'apomixie, on laisse la plante produire des semences et on recueille parmi les semences des semences haploïdes viables.

2. Procédé selon la revendication 1 de production d'haploïdes doublés chez un angiosperme qui comprend les étapes ultérieures de culture de la semence haploïde dans un environnement qui favorise le développement de l'embryon en une plante qui est capable de produire des semences haploïdes doublées.

3. Procédé selon la revendication 1 de production de semences haploïdes génétiquement stables, sources d'une lignée de plantes homozygotes qui comprend les étapes ultérieures de croissance des semences haploïdes dans un environnement qui favorise le développement de son embryon en une plante mûre productrice de semences où chaque semençe est génétiquement stable et représente la source d'une lignée homozygote.

4. Procédé de la revendication 3 dans lequel les lignées de plantes homozygotes sont une graine de céréale ou une culture fourragère, on applique l'agent apomixique en une quantité allant de 0,03 à 11 kilogrammes par hectare et on l'applique à la plante à un moment situé entre la survenue de la méïose et de l'anthèse.

5. Procédé selon la revendication 3 ou 4 qui comprend l'étape ultérieure de culture d'une lignée de plante homozygote à partir de la semence produite par les plantes haploïdes doublées.

6. Procédé des revendications 1, 2 ou 3 dans lequel on applique l'agent apomixique en une quantité de 0,01 à 22 kilogrammes par hectare.

7. Procédé de la revendication 6 dans lequel on applique l'agent apomixique à une plante monocotylédone à un moment qui se situe entre la méïose et l'anthèse.

8. Procédé de la revendication 6 dans lequel l'agent apomixique comprend un ou plusieurs des suivants: Acide 1 - (4 - chloro-phényl) - 1,4 - dihydro - 4 - oxo - 6 - méthylpyridazine - 3 - carboxylique, Acide 1 - (4 - chlorophényl) - 1,4 - dihydro - 4 - oxo - 5 - carboxyméthyl - 6 - éthylpyridazine - 3 - carboxylique, Acide 1 - (4 - chloro-phényl) - 1,4 - dihydro - 4 - oxo - 5 - propionyl - 6 - méthylpyridazine - 3 - carboxylique, Acide 1 - éthyl - 6 - méthyl - 2 - (4 - chlorophényl) - 4 - oxonicotinique, Acide 1 - éthyl - 6 - méthyl - 2 - (3,4 - dichloro-phényl) - 4 - oxonicotinique, 1 - (3,4 - dichloro-phényl) - 1,4 - dihydro - 4 - oxo - 5 - carboxyméthyl - 6 - méthylpyridazine et 1 - phényl - 1,4 - dihydro - 4 - oxo - 5 - carboxyméthyl - 6 - éthylpyridazine, et leurs sels solubles dans l'eau et 1 - (4 - chlorophényl) - 1,4 - dihydro - 4 - oxo - 6 - éthylpyridazine - 5 - carboxylate de méthyle ou d'éthyle et 1 - (4 - bromophényl) - 1,4 - dihydro - 4 - oxo - 6 - éthylpyridazine - 5 - carboxylate de méthyle ou d'éthyle.

9. Procédé de la revendication 8 dans lequel on applique l'agent apomixique en une quantité de 0,03 à 11 kilogrammes par hectare.

10. Procédé pour induire une apomixie chez une plante angiosperme dans lequel on applique à la plante à un moment compris entre le début de la floraison et l'anthèse et en une quantité suffisante pour provoquer l'apomixie, un agent apomixique qui comprend un ou plusieurs des 4-oxo(thio)nicotinates et 4-oxo(thio)pyridazines qui ont des propriétés de régulation de la croissance.

11. Procédé de la revendication 10 dans lequel l'agent apomixique est un agent hybridant chimique efficace par rapport à la plante à laquelle il est appliqué.

12. Procédé selon la revendication 1 ou 2 dans lequel la plante comprend une graine de céréale ou une plante de culture fourragère et l'agent apomixique est appliqué à un moment situé entre la méïose et juste avant l'anthèse et en une quantité de 0,03 à 11 kilogrammes par hectare.

13. Procédé selon la revendication 2 dans lequel la plante comprend une graine de céréale ou une plante de culture fourragère et l'agent apomixique est appliqué à un moment situé entre la méïose et juste avant l'anthèse en une quantité de 0,03 à 11 kilogrammes par hectare.

14. Procédé de la revendication 10 ou 13 dans lequel l'agent apomixique comprend un ou plusieurs 4-oxo(thio)nicotinates ou 4-oxo(thio)pyridazines des structures suivantes:

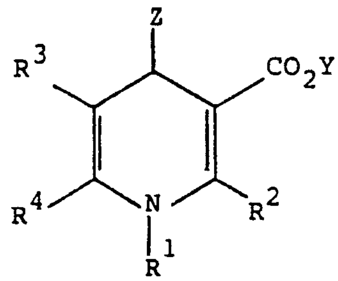

où

Z est un atome d'oxygène ou de soufre;

$R^1$ est un alcoyle en $C_1$ à $C_6$ ou alcényle en $C_2$ à $C_6$ éventuellement substitué; au moins l'un des radicaux $R^2$, $R^3$ ou $R^4$ est un

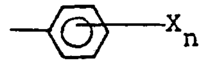

("le groupe phényle") où

lorsque $R^2$ est "le groupe phényle", $R^3$ est un atome d'hydrogène, un groupe alcoyle ou un atome d'halogène et $R^4$ est un atome d'hydrogène, un groupe alcoyle ou représente indépendamment le groupe phényle;

lorsque $R^3$ est "le groupe phényle", $R^2$ est un groupe alcoyle ou représente indépendamment "le groupe phényle", et $R^4$ est un atome d'hydrogène ou un groupe alcoyle;

lorsque $R^4$ est "le groupe phényle", $R^2$ est un groupe alcoyle en $C_2$ à $C_6$ éventuellement substitué, un groupe alcényle en $C_3$ à $C_6$ ou représente indépendamment "le groupe phényle" et $R^3$ est un atome d'hydrogène, un groupe alcoyle en $C_1$ à $C_6$ ou un atome d'halogène; et

Y est un atome d'hydrogène, un de ses sels agronomiquement acceptables ou un groupe alcoyle;

X est un atome d'hydrogène, un atome d'halogène, un groupe trihalométhyle, un groupe alcoyle en $C_1$ à $C_6$, un groupe nitro, un groupe cyano ou un groupe alcoxy en $C_1$ à $C_6$; et

n est un nombre entier allant de 1 à 3; et

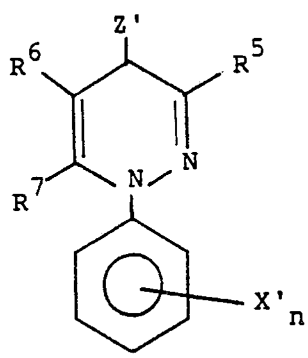

où

Z' est un atome d'oxygène ou de soufre;

$R^5$ est un atome d'hydrogène, un atome d'halogène,

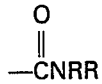

où R et R représentent chacun indépendamment un hydrogène, alcoyle, alcoyle substitué par un groupe carbonyle ou carboxy,

$$-\overset{\overset{O}{\|}}{C}WR^8$$

où W est un atome d'oxygène ou de soufre et $R^8$ est un atome d'hydrogène, un groupe alcoyle, aryloxy, alcoxyalcoyle, alcoylhalogène, alcoylhydroxy, iminoalcoyle, aryle, alcényle, cyclo-alcoyle en $C_3$ à $C_6$ ou cycloalcoyl-alcoyle;

$R^6$ est un atome d'hydrogène, un alcoyle,

$$-\overset{\overset{O}{\|}}{C}A$$

où A est un hydrogène, alcoyle, aralcoyle, hydroxyle, alcoxy, thio, alcoylthio ou $—NR^9R^{10}$ où $R^9$ et $R^{10}$ représentent chacun indépendamment un hydrogène, alcoyle, alcoyle substitué par un groupe carbonyle ou carboxy, à condition que $R^5$ et $R^6$ ne soient pas tous deux des atomes d'hydrogène; et lorsque $R^5$ est un atome d'hydrogène ou d'halogène, alors $R^6$ représente

$$-\overset{\overset{O}{\|}}{C}A'$$

ou A' est un groupe alcoxy, thioalcoyle ou $—NR^9R^{10}$; et

$R^7$ est un alcoyle, aryle ou aryle substitué par jusqu'à trois substituants choisis dans le groupe constitué par halogène, nitro, trihalométhyle, alcoxy en $C_1$ à $C_4$, alcoyle en $C_1$ à $C_4$ ou cyano;

n vaut 0, 1, 2 ou 3;

chaque radical X' représente indépendamment un atome d'hydrogène, un atome d'halogène, un group trihalométhyle, alcoyle, alcoxy, cyano ou nitro; et de leurs sels agronomiquement acceptables.

15. Procédé de la revendication 14 dans lequel:

Z et Z' sont des atomes d'oxygène;

X et X' représentent indépendamment un hydrogène, un halogène ou un groupe trifluorométhyle;

n vaut 1 ou 2;

$R^1$ est un groupe alcoyle en $C_1$ à $C_4$;

$R^2$ ou $R^4$ est "le groupe phényle";

lorsque $R^2$ est "le groupe phényle", $R^3$ est un alcoyle en $C_1$ à $C_3$, un atome de brome ou un atome d'hydrogène; $R^4$ est un atome d'hydrogène, un atome d'halogène ou un groupe alcoyle en $C_1$ à $C_4$;

lorsque $R^4$ est "le groupe phényle", $R^2$ est un atome d'hydrogène, un atome d'halogène ou un groupe alcoyle en $C_1$ à $C_4$; $R^3$ est un atome d'halogène ou un atome d'hydrogène;

Y est un atome d'hydrogène, un alcoyle en $C_1$ à $C_6$ ou un cation de métal alcalin ou de métal alcalinoterreux;

$R^5$ est un hydrogène, halogène ou

$$-\overset{\overset{O}{\|}}{C}WR^8$$

où W est un atome d'oxygène et $R^8$ est un atome d'hydrogène, un alcoyle en $C_1$ à $C_4$, alcoyle en $C_1$ à $C_4$-halogène, alcoyle en $C_1$ à $C_4$-hydroxy, alcoxy en $C_1$ à $C_4$-alcoyle en $C_1$ à $C_4$, phényle en $C_1$ à $C_2$, alcényle en $C_1$ à $C_4$ ou iminoalcoyle en $C_1$ à $C_4$ ou W est un soufre et $R^5$ est un groupe alcoyle en $C_1$ à $C_4$;

$R^6$ est un hydrogène, alcoyle en $C_1$ à $C_4$,

$$-\overset{\overset{O}{\|}}{C}A$$

où A est un alcoyle en $C_1$ à $C_4$, alcoxy en $C_1$ à $C_4$, alcoylthio en $C_1$ à $C_4$ ou $—NR^9R^{10}$ et lorsque $R^5$ est un hydrogène ou un halogène, alors $R^6$ est

$$-\overset{\overset{O}{\|}}{C}A$$

et A est un alcoxy en $C_1$ à $C_4$ ou alcoylthio en $C_1$ à $C_4$;

$R^7$ est un groupe alcoyle en $C_1$ à $C_4$; et leurs sels agronomiquement acceptables.

16. Procédé de la revendication 15 dans lequel:

Z et Z' sont des atomes d'oxygène;

X est un atome d'hydrogène, un chloro, bromo, fluoro ou iodo;

X' est un chloro, fluoro ou iodo;

n vaut 1 ou 2;

$R^1$ est un groupe méthyle, éthyle ou *n*-propyle;

$R^2$ est un groupe alcoyle en $C_1$ à $C_4$;

$R^3$ est un atome d'hydrogène;

$R^4$ est "le groupe phényle";

Y est un cation sodium ou potassium;

$R^5$ est

$$-\overset{\overset{\displaystyle O}{\|}}{C}WR^8,$$

où W est un atome d'oxygène et $R^8$ est un atome d'hydrogène ou un de ses sels de sodium, potassium ou triéthanolamine, ou un groupe alcoyle en $C_1$ à $C_2$;

$R^6$ est un atome d'hydrogène ou

$$-\overset{\overset{\displaystyle O}{\|}}{C}A'$$

où A' est un groupe hydroxy ou alcoxy en $C_1$ à $C_2$;

$R^7$ est un groupe méthyle, éthyle ou *n*-propyle; et leurs sels agronomiquement acceptables.

17. Procédé de la revendication 12 ou 13 dans lequel la culture est le blé, l'orge, l'avoine, le riz, le seigle, le maïs, le sorgho, le millet ou le triticale, l'agent apomixique est appliqué en une quantité de 0,1 à 6 kilogrammes par hectare et l'agent apomixique comprend un ou plusieurs des composés: Acide 1 - (4 - chloro-phényl) - 1,4 - dihydro - 4 - oxo - 6 - méthylpyridazine - 3 - carboxylique, Acide 1 - (4 - chlorophényl) - 1,4 - dihydro - 4 - oxo - 5 - carboxyméthyl - 6 - méthylpyridazine - 3 - carboxylique, Acide 1 - (4 - chlorophényl) - 1,4 - dihydro - 4 - oxo - 5 - propionyl - 6 - méthyl-pyridazine - 3 - carboxylique, Acide 1 - éthyl - 6 - méthyl - 2 - (4 - chlorophényl) - 4 - oxonicotinique, Acide 1 - éthyl - 6 - méthyl - 2 - (3,4 - dichlorophényl) - 4 - oxonicotinique, 1 - (3,4 - dichlorophényl) - 1,4 - dihydro - 4 - oxo - 5 - carboxyméthyl - 6 - méthylpyridazine et 1 - phényl - 1,4 - dihydro - 4 - oxo - 5 - carboxyméthyl - 6 - éthylpyridazine, et leurs sels solubles dans l'eau et 1 - (4 - chlorophényl) - 1,4 - dihydro - 4 - oxo - 6 - éthylpyridazine - 5 - carboxylate de méthyle ou d'éthyle et 1 - (4 - bromophényl) - 1,4 - dihydro - 4 - oxo - 6 - éthylpyridazine - 5 - carboxylate de méthyle ou d'éthyle.